# EUROPEAN PATENT SPECIFICATION

(11) **EP 3 600 148 B1**
(45) Date of publication and mention of the grant of the patent: **07.12.2022**
(21) Application number: 18715139.4
(22) Date of filing: 21.03.2018
(51) Int. Cl.: A61F 2/07

(54) **STENT-GRAFTS FOR SEALING AROUND EXTERNAL DISTURBANCES**
STENTTRANSPLANTATE ZUR ABDICHTUNG UM EXTERNE STÖRUNGEN
STENT-GREFFONS POUR ASSURER L'ÉTANCHÉITÉ AUTOUR DE PERTURBATIONS EXTERNES

(30) Priority: 21.03.2017 US 201762474391 P
(43) Date of publication of application: 05.02.2020
(73) Proprietor: Endospan Ltd., 46733 Herzilyia Pituach (IL)
(72) Inventor: ZIGELBOIM, Or, Ness Ziona (IL); MARMUR, Yaniv, 20600 Yokneam Moshava (IL); SHALEV, Alon, 4331513 Ra'anana (IL)
(74) Representative: Evens, Paul Jonathan
(86) International application number: PCT/IL2018/050325
(87) International publication number: WO 2018/173056

(56) References cited:
- WO-A1-2014/197743
- WO-A2-00/42949
- US-B1- 6 729 356

## Description

### FIELD OF THE APPLICATION

The present invention relates generally to implantable medical devices, and specifically to implantable stent-grafts.

### BACKGROUND OF THE APPLICATION

Endovascular prostheses are sometimes used to treat aortic aneurysms. Such treatment includes implanting a stent or stent-graft within the diseased vessel to bypass the anomaly. An aneurysm is a sac formed by the dilation of the wall of the artery. Aneurysms may be congenital, but are usually caused by disease or, occasionally, by trauma. Aortic aneurysms which commonly form between the renal arteries and the iliac arteries are referred to as abdominal aortic aneurysms ("AAAs"). Other aneurysms occur in the aorta, such as thoracic aortic aneurysms ("TAAs") and aortic uni-iliac ("AUI") aneurysms. A TAA may occur downstream the aortic arch, i.e., in the descending aorta. Alternatively, a TAA may occur in the aortic arch itself, where the aorta branches to supply the brachiocephalic, left carotid and subclavian arteries, or may occur in the ascending aorta.

Endo-Vascular Aneurysm Repair (EVAR) has transformed the practice of treatment of aortic aneurysms from an open surgical approach to a much less invasive surgical approach. The first step of an endovascular intervention usually requires introducing a delivery system into the vasculature of a subject. If the crossing profile, i.e., the external diameter, of the delivery system is 24 Fr or lower (3 Fr = 1 millimeter), a true percutaneous approach may be used, because vascular closure devices are available for proper closure of such puncture sites.

Blood vessels occasionally weaken or even rupture. For example, in the aortic artery, the vascular wall can weaken or tear, resulting in dangerous conditions such as aneurysm and dissection. Treatment of such conditions can be performed by implanting a stent-graft within the vascular system using minimally-invasive surgical procedures. An endoluminal stent-graft typically includes one or more stents affixed to graft material and is delivered to the treatment site by endovascular insertion. Once the endoluminal stent-graft is radially enlarged, it should remain in place indefinitely by self-attachment to the vessel wall, acting as a substitute vessel for the flow of blood or other fluids.

Aortic dissection is a tear or partial tear in the inner wall of the aorta, which causes blood to flow between the layers of the wall of the aorta, forcing the layers apart. Aortic dissections may be divided into two types in accordance with the Stanford classification. Type A dissections involve the ascending aorta and/or aortic arch, and possibly the descending aorta. Type B dissections involve the descending aorta or the arch (distal to right brachiocephalic artery origin), without involvement of the ascending aorta.

WO 2014/197743 relates to variable depression stents, and discloses apparatus according to the pre-characterizing portion of appended claim 1.

### SUMMARY OF THE APPLICATION

In accordance with the present invention, there is provided apparatus as defined in appended independent claim 1. Embodiments of the present invention are defined in appended claims which depend on independent claim 1. Methods of using the apparatus are described hereinafter to better understand the apparatus, but are not within the scope of the present invention.

Embodiments of the present invention provide an endovascular stent-graft, which comprises struts and a fluid flow guide, which is fixed to first and second subsets of the struts. The struts of the first and the second subsets are elastic such that, when the stent-graft assumes the radially-expanded state:
- the fluid flow guide defines a lumen having a central longitudinal axis,
- the struts of the first subset cause the fluid flow guide to define a plurality of substantially cylindrical tubular portions, and
- the struts of the second subset cause the fluid flow guide to define a bulge having a greatest bulge radius from the central longitudinal axis, which greatest bulge radius is at least 5% greater than an average radius of the substantially cylindrical tubular portions proximally and distally adjacent the bulge.

For some applications, the bulge is configured to reduce the likelihood of long-term leakage (i.e., blood flow) through gutters, i.e., the residual intravascular space disposed outside the lumens of the endovascular stent-graft and branching stent-grafts disposed alongside the endovascular stent-graft. Alternatively or additionally, the bulge may reduce the likelihood of blood flow between the endovascular stent-graft and features of the anatomy of the blood vessel wall, such as isolated regions of plaque, calcifications, or thrombus, all of which alter the circularity of the blood vessel wall and might otherwise present issues for good sealing between the stent-graft and the blood vessel wall. As a result, the likelihood of type 1 endoleak is reduced.

For some applications, the struts of the second subset have an average wall thickness, measured radially, that is no more than 80% (e.g., no more than 60%) of an average wall thickness of the struts of the first subset, measured radially. Such a lower wall thickness may contribute to a lower spring constant of the struts of the second subset than the struts of the first subset, which may facilitate more local deformation (indentation) of the bulge around a branching stent-graft, without unnecessarily crushing the branching stent-graft, or necessitating internally reinforcement of the branching stent-graft with additional metallic stents.

The struts of the first subset are arranged as a plurality of circumferential cells in circumferentially-continuous rings, which cause the fluid flow guide to define the plurality of substantially cylindrical tubular portions. The struts of the second subset define tip portions. The number of tip portions of the struts of the second subset that define the bulge is at least 50% greater than the average number of the circumferential cells in the two the circumferentially-continuous rings proximally and distally adjacent the bulge, such as least 175% greater, no more than 250% greater, and/or between 175% and 250% (e.g., 200%) of the average number of the circumferential cells. Providing such a relatively large number of tip portions may facilitate more local deformation (indentation) of the bulge around a branching stent-graft, without unnecessarily crushing the branching stent-graft, or necessitating internally reinforcement of the branching stent-graft with additional metallic stents.

There is therefore provided, in accordance with an Inventive concept 1 of the present invention, apparatus for use with a delivery catheter, the apparatus including an endovascular stent-graft, which is configured to initially be positioned in the delivery catheter in a radially-compressed state, and to assume a radially-expanded state upon being deployed from the delivery catheter, and which includes:
struts; and
a fluid flow guide, which includes at least one biologically-compatible substantially blood-impervious fabric, and which is fixed to first and second subsets of the struts, wherein the first and the second subsets do not include any common struts,
wherein the struts of the first and the second subsets are elastic such that, when the stent-graft assumes the radially-expanded state:
   the fluid flow guide defines a lumen having a central longitudinal axis,
   the struts of the first subset are arranged as a plurality of circumferential cells in circumferentially-continuous rings, which cause the fluid flow guide to define a plurality of substantially cylindrical tubular portions,
   the struts of the second subset cause the fluid flow guide to define a bulge having a greatest bulge radius from the central longitudinal axis, which greatest bulge radius is at least 5% greater than an average radius of the substantially cylindrical tubular portions proximally and distally adjacent the bulge, characterized in that:
      the struts of the second subset define tip portions, and
      the number of the tip portions of the struts of the second subset that define the bulge is at least 50% greater than the average number of circumferential cells in the two circumferentially-continuous rings proximally and distally adjacent the bulge.

In one embodiment, the number of the tip portions of the struts of the second subset that define the bulge may equal between 175% and 250% of the average number of circumferential cells. For example, the number of the tip portions of the struts of the second subset that define the bulge may equal 200% of the average number of circumferential cells.

In one embodiment, the number of the tip portions of the struts of the second subset that define the bulge may equal between 12 and 16.

In one embodiment, the bulge may completely circumferentially encircle the stent-graft.

In one embodiment, the tip portions may be directly sutured to the fluid flow guide.

In one embodiment, the tip portions may be shaped so as to define respective atraumatic features.

In one embodiment, the fluid flow guide may be arranged such that all of the fabric defines the lumen when the stent-graft assumes the radially-expanded state.

In one embodiment, the circumferentially-continuous rings may undulate, such that the circumferential cells are shaped so as to define respective peaks and troughs, wherein the peaks and troughs are substantially semicircular, have a first average radius of curvature, and are shaped so as to define respective strain-distribution features having a second average radius of curvature equal to between 10% and 50% of the first average radius of curvature.

In one arrangement, an axial length of the bulge may equal between 10% and 40% of a difference between (a) the greatest bulge radius and (b) the average radius of the substantially cylindrical tubular portions proximally and distally adjacent the bulge.

In one arrangement, the struts of the first and the second subsets may be arranged and shaped such that the struts of the first subset apply a radially-outward force that is at least 20% greater than a radially-outward applied by the struts of the second subset. For example, the struts of the first and the second subsets may be arranged and shaped such that the struts of the first subset apply the radially-outward force that is at least 40% greater than the radially-outward applied by the struts of the second subset.

In one arrangement, the circumferential cells may be W-shaped.

In one arrangement, the struts of the first and the second subsets may be superelastic.

In one arrangement, the circumferential cells may be diamond-shaped.

In one embodiment,
the bulge may be a first bulge,
the fluid flow guide may be fixed to the first subset of the struts, the second subset of the struts, and a third subset of the struts so as to define the lumen, wherein the first, the second, and the third subsets do not include any common struts, and
the struts of the third subset may be elastic such that, when the stent-graft assumes the radially-expanded state:
   the struts of the third subset cause the fluid flow guide to define a second bulge having a greatest bulge radius from the central longitudinal axis, which greatest bulge radius is at least 5% greater than an average radius of the substantially cylindrical tubular portions proximally and distally adjacent the second bulge,
   the struts of the third subset define tip portions, and
   the number of the tip portions of the struts of the third subset that define the second bulge is at least 50% greater than the average number of circumferential cells in the two circumferentially-continuous rings proximally and distally adjacent the second bulge.

In one embodiment, at least a first one of the struts of the second subset may be structurally integral with at least a second one of the struts of the first subset. For example, all of the struts of the second subset may be structurally integral with at least one of the struts of the first subset,
none of the struts of the second subset may be directly connected to any of the other struts of the second subset, and
none of the struts of the second subset may be indirectly connected to any of the other struts of the second subset by any struts other than the struts of the first subset.

In one arranement, for at least a first one of the tip portions of first one of the struts of the second subset, an angle may be defined by (a) the central longitudinal axis and (b) a line defined by (i) the first one of the tip portions and (ii) a junction between the first one of the struts of the second subset and the second one of the struts of the first subset, and the angle may be between 5 and 60 degrees. For example, the angle may be between 10 and 30 degrees.

In one arrangement,
the stent-graft may further include sutures that secure the struts of the first and the second subsets to the fluid flow guide, and
for at least a first one of the tip portions of the first one of the struts of the second subset:
   the first one of the struts of the second subset may have a length measured along the first one of the struts between (a) the first tip portion and (b) a junction between the first one of the struts of the second subset and the second one of the struts of the first subset,
   a far half of the first one of the struts may extend from the first tip portion along 50% of the length of the first one of the struts of the second subset, and
   none of the sutures may be disposed along at least 50% of the far half of the first one of the struts of the second subset.

In one embodiment,
the struts of the first subset may be arranged in a plurality of undulating circumferentially-continuous rings having alternating peaks and troughs, and
a first plurality of the struts of the second subset may originate in a proximal half of one of the undulating circumferentially-continuous rings, and a second plurality of the struts of the second subset may originate in a distal half of the one of the undulating circumferentially-continuous rings.

In one arrangement, the first plurality of the struts of the second subset may originate in a proximal 20% of an axial height of the one of the undulating circumferentially-continuous rings. For example, the first plurality of the struts of the second subset may originate at proximal-most sites of the one of the undulating circumferentially-continuous rings. Also for example, the second plurality of the struts of the second subset may originate in a distal 20% of the axial height of the one of the undulating circumferentially-continuous rings.

In one embodiment,
the first plurality of the struts of the second subset may originate at proximal-most sites of the one of the undulating circumferentially-continuous rings, and
the second plurality of the struts of the second subset may originate at distal-most sites of the one of the undulating circumferentially-continuous rings.

In one arrangement, a first plurality of the struts of the second subset may originate at two or more axially different locations of one of the undulating circumferentially-continuous rings.

In one arrangement, at least one of the struts of the second subset may not be structurally integral with any of the struts of the first subset.

In one embodiment,
the fluid flow guide may be fixed to the first subset of the struts, the second subset of the struts, and a third subset of the struts so as to define the lumen, wherein the first, the second, and the third subsets do not include any common struts, and
the struts of the third subset may be elastic such that, when the stent-graft assumes the radially-expanded state, the struts of the third subset cause the fluid flow guide to define a flared axial portion that extends to one end of the fluid flow guide, the flared axial portion having (a) a greatest flared radius from the central longitudinal axis, which greatest flared radius is at least 5% greater than a radius of the substantially cylindrical tubular portion axially adjacent the flared axial portion, and (b) an axial length equal to between 5% and 20% of a difference between (i) the greatest flared radius and (ii) the radius of the substantially cylindrical tubular portion axially adjacent the flared axial portion.

In one arrangement,
the flared axial portion may be a first flared axial portion, and the one end of the fluid flow guide is a first end of the fluid flow guide, and
the struts of the third subset may be elastic such that, when the stent-graft assumes the radially-expanded state, the struts of the third subset cause the fluid flow guide to additionally define a second flared axial portion that extends to a second end of the fluid flow guide, the second flared axial portion having (a) a greatest flared radius from the central longitudinal axis, which greatest flared radius is at least 5% greater than an average radius of the substantially cylindrical tubular portion axially adjacent the second flared axial portion, and (b) an axial length equal to between 5% and 20% of a difference between (i) the greatest flared radius and (ii) the radius of the substantially cylindrical tubular portion axially adjacent the second flared axial portion.

In one arrangement,
the stent-graft may further include sutures that secure the struts of the first and the second subsets to the fluid flow guide, and
at least 80% of a surface area of the struts of the first subset may be within 3 mm of at least one of the sutures that secure the struts of the first subset to the fluid flow guide. For example,
   no more than 50% of the surface area of the struts of the second subset may be within 3 mm of at least one of the sutures that secure the struts of the second subset to the fluid flow guide.

In one arrangement, respective circumferences of all substantially cylindrical tubular portions of the fluid flow guide may vary by less than 10%.

In one arrangement, a circumference of a first one of the substantially cylindrical tubular portions may be at least 10% greater than a circumference of a second one of the substantially cylindrical tubular portions.

In one embodiment, when the endovascular stent-graft is removably disposed in the delivery catheter in the radially-compressed state, the struts of the first subset may not coincide with the struts of the second subset.

In one arrangement,
the stent-graft may be a main stent-graft,
the fluid flow guide may not be shaped so as to define any fenestrations or scallops, and
the apparatus may further include one or more branching stent-grafts.

In one arrangement,
the stent-graft may be a main stent-graft,
the fluid flow guide may not be shaped so as to define one or more openings selected from the group of openings consisting of: fenestrations, scallops, and fenestrations and scallops, and
the apparatus may further include a number of branching stent-grafts, the number greater than a number of the openings.

The present invention will be more fully understood from the following detailed description of embodiments thereof, taken together with the drawings, in which:

### BRIEF DESCRIPTION OF THE DRAWINGS

Figs. 1A-C are schematic illustrations of an endovascular stent-graft, in accordance with an application of the present invention;
Figs. 2A-D are schematic illustration of exemplary deployments of an endovascular system in an aneurysmal descending aorta, in accordance with respective applications of embodiments of the present invention;
Fig. 3 is a schematic illustration of cutting pattern for the fabric of a fluid flow guide of the endovascular stent-graft of Figs. 1A-C during fabrication;
Fig. 4 is a schematic illustration of a single undulating circumferentially-continuous ring of the endovascular stent-graft of Figs. 1A-C;
Figs. 5A-B are schematic illustrations of alternative configurations of a single undulating circumferentially-continuous ring of the endovascular stent-graft of Figs. 1A-C;
Fig. 6 is a schematic illustration of another endovascular stent-graft, in accordance with an application of the present invention;
Fig. 7 is a schematic illustration of an exemplary deployment of an endovascular system in an aneurysmal descending aorta, in accordance with an application of an embodiment of the present invention;
Fig. 8 is a schematic illustration of yet another endovascular stent-graft, in accordance with an application of the present invention;
Figs. 9A-B are schematic illustrations of still another endovascular stent-graft, in accordance with an application of the present invention;
Figs. 10A-B are schematic illustrations of a single undulating circumferentially-continuous ring of an endovascular stent-graft when the stent-graft assumes a radially-compressed delivery state and a radially-expanded state, respectively; and
Figs. 11A-B are schematic illustrations of another endovascular stent-graft.

### DETAILED DESCRIPTION OF APPLICATIONS

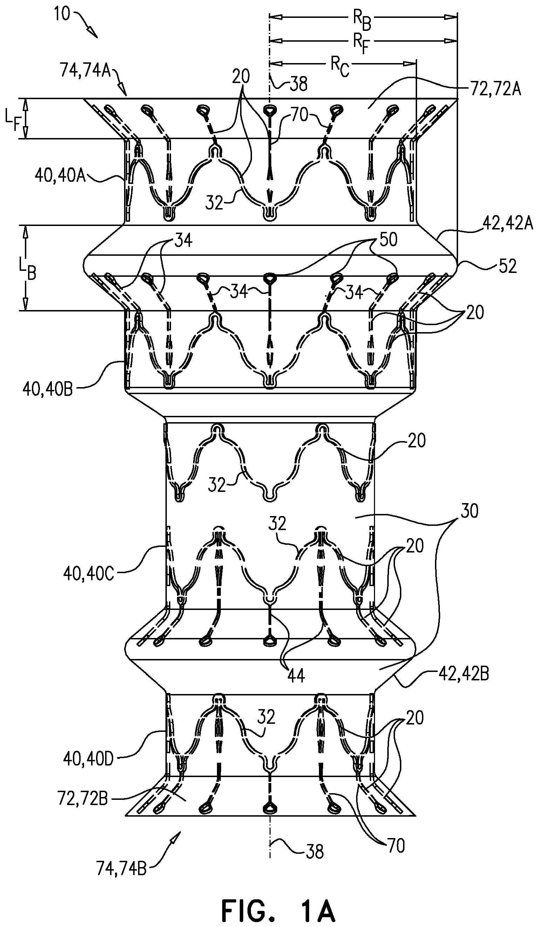

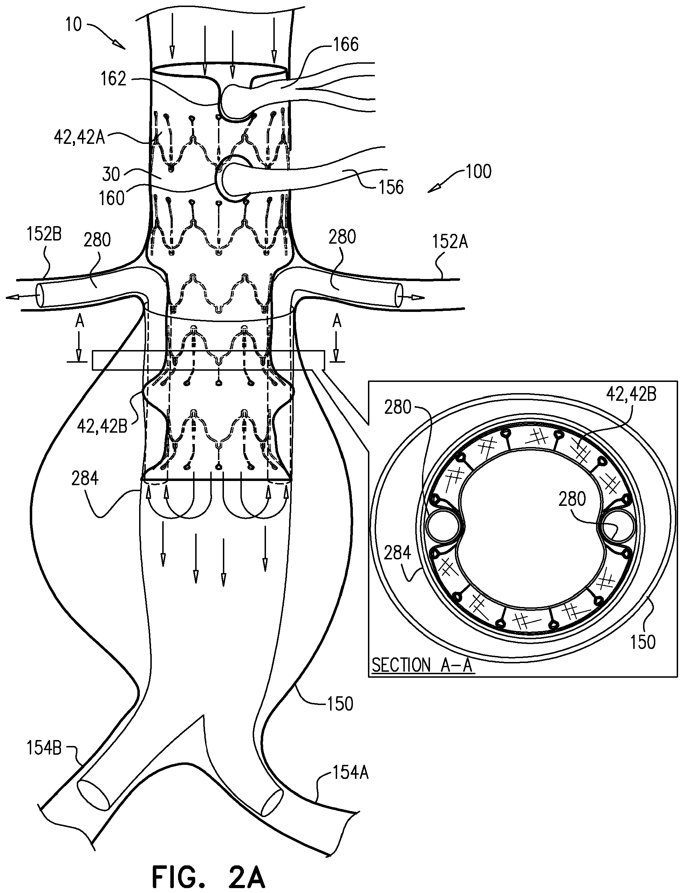

Figs. 1A-C are schematic illustrations an endovascular stent-graft 10, in accordance with an application of the present invention. Endovascular stent-graft 10 may be provided as part of an endovascular system, which may additionally comprise other components, such as an endovascular delivery tool (e.g., comprising a delivery catheter), and/or additional endovascular stent-grafts, such as described hereinbelow with reference to Figs. 2A-D. The endovascular system may be used to treat a blood vessel, such as an artery, e.g., descending aorta 150, suffering from an aneurysm, a dissection, or, more generally, a pathologically dilated blood vessel.

Endovascular stent-graft 10 is configured to initially be positioned in the delivery catheter in a radially-compressed state, and to assume a radially-expanded state upon being deployed from the delivery catheter, such as shown in Figs. 1A-C and 2A-D. For some applications, endovascular stent-graft 10 is self-expanding, i.e., is configured to automatically transition from the radially-compressed delivery state to the radially-expanded state upon being released from the delivery catheter. For other applications, stent-graft 10 is plastically expandable, such as balloon-expandable.

Endovascular stent-graft 10 comprises:
- struts 20 (i.e., elongate segments that provide structure to stent-graft 10, and are optionally interconnected with one another at respective junctions; optionally, a plurality of struts 20 are fabricated from a cylindrical tube, such as by laser cutting); and
- a fluid flow guide 30, which comprises at least one biologically-compatible substantially blood-impervious fabric, and which is fixed to first and second subsets 32 and 34 of struts 20; first and second subsets 32 and 34 do not include any common struts 20.

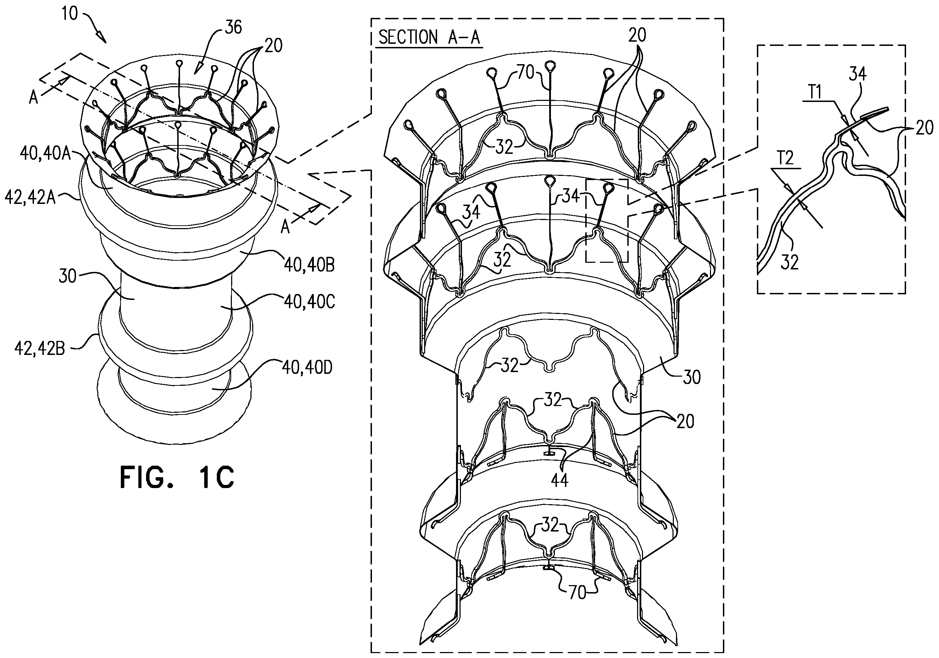

Struts 20 of first and second subsets 32 and 34 are elastic such that, when stent-graft 10 assumes the radially-expanded state, such as shown in Figs. 1A-C:
- fluid flow guide 30 defines a lumen 36 (labeled in Fig. 1C) having a central longitudinal axis 38 (labeled in Figs. 1A-B),
- struts 20 of first subset 32 cause fluid flow guide 30 to define a plurality of substantially cylindrical tubular portions 40, and
- struts 20 of second subset 34 cause fluid flow guide 30 to define a bulge 42 having a greatest bulge radius R_{B} from central longitudinal axis 38, which greatest bulge radius R_{B} is at least 5% greater (e.g., at least 10% greater, such as at least 15% greater) than an average radius R_{C} of substantially cylindrical tubular portions 40A and 40B proximally and distally adjacent bulge 42 (i.e., axially surrounding bulge 42).

For some applications, bulge 42 is configured to reduce the likelihood of long-term leakage (i.e., blood flow) through gutters, i.e., the residual intravascular space disposed outside the lumens of endovascular stent-graft 10 and branching stent-grafts disposed alongside endovascular stent-graft 10. Alternatively or additionally, bulge 42 may reduce the likelihood of blood flow between endovascular stent-graft 10 and features of the anatomy of the blood vessel wall, such as isolated regions of plaque, calcifications, or thrombus, all of which alter the circularity of the blood vessel wall and might otherwise present issues for good sealing between the stent-graft and the blood vessel wall. As a result, the likelihood of type 1 endoleak is reduced.

For some applications, an axial length L_{B} (labeled in Fig. 1A) of bulge 42 equals at least 10%, no more than 40%, and/or between 10% and 40% of a difference between (a) the greatest bulge radius R_{B} and (b) the average radius R_{C} of substantially cylindrical tubular portions 40A and 40B proximally and distally adjacent bulge 42 (i.e., axially surrounding bulge 42).

As used in the present application, including in the claims, the "central longitudinal axis" 38 of lumen 36 is the set of all centroids of transverse cross-sectional sections of lumen 36 along lumen 36. Thus the cross-sectional sections are locally perpendicular to central longitudinal axis 38, which runs along lumen 36. (For configurations in which lumen 36 is circular in cross-section, the centroids correspond with the centers of the circular cross-sectional sections.)

Typically, struts 20 comprise a metal, such as a flexible metal, an elastic metal, stainless steel (e.g., elastic stainless steel), cobalt-chromium, or a superelastic alloy (such as Nitinol). Fluid flow guide 30 comprises at least one biologically-compatible substantially blood-impervious fabric (e.g., one or more thin flexible sheets), which are typically arranged as a tubular structure (with a diameter that varies therealong), when stent-graft 10 is unconstrained in the radially-expanded state. The fabric may comprise, for example, a polymeric material (e.g., a polyester, or polytetrafluoroethylene (PTFE)), a textile material (e.g., polyethylene terephthalate (PET), e.g., Dacron^{®}, manufactured by E. I. du Pont de Nemours and Company, Wilmington, DE, USA), or expanded polytetrafluoroethylene (ePTFE), e.g., manufactured by W. L. Gore & Associates, Newark, DE, USA), natural tissue (e.g., saphenous vein or collagen), or a combination thereof.

Typically, fluid flow guide 30 is arranged such that all of the fabric defines lumen 36 when stent-graft 10 assumes the radially-expanded state (such that, for example, stent-graft 10 does not comprise any skirts that extend radially outward from fluid flow guide 30).

For some applications, struts 20 of first and second subsets 32 and 34 are arranged and shaped such that struts 20 of first subset 32 apply a radially-outward force that is at least 20% (e.g., at least 40%) greater than a radially-outward applied by struts 20 of second subset 34.

For some applications, as shown in Figs. 1A-C, fluid flow guide 30 is shaped so as to define one or more additional bulges 42. For some applications, bulge 42 is a first bulge 42A, and fluid flow guide 30 is fixed to first subset 32 of struts 20, second subset 34 of struts 20, and a third subset 44 of struts 20 so as to define lumen 36; first subset 32, second subset 34, and third subset do not include any common struts 20. Struts 20 of third subset 44 are elastic such that, when stent-graft 10 assumes the radially-expanded state, struts 20 of third subset 44 cause fluid flow guide 30 to define a second bulge 42B having a greatest bulge radius from central longitudinal axis 38, which greatest bulge radius is at least 5% greater than an average radius of substantially cylindrical tubular portions 40C and 40D proximally and distally adjacent second bulge 42B (i.e., axially surrounding bulge 42B). First bulge 42A may prevent leakage from a chimney (i.e., from above), and second bulge 42B may prevent retrograde leakage from a "periscope" (i.e., from below), such as described hereinbelow with reference to Figs. 2A-D.

For some applications, bulge 42 completely circumferentially encircles stent-graft 10. For other applications, bulge 42 only partially circumferentially encircles stent-graft 10. For some applications in which fluid flow guide 30 defines a plurality of bulges, along an entire axial length of fluid flow guide 30, each circumferential angle is at least circumscribed by one bulge 42. For some applications in which fluid flow guide 30 defines a plurality of bulges, along an entire axial length of fluid flow guide 30, each circumferential angle is at least circumscribed by two bulges 42.

At least a first one of struts 20 of second subset 34 defines a first tip portion 50. For some applications, an angle is defined by (a) central longitudinal axis 38 and (b) a line defined by (i) the first tip portion 50 and (ii) a junction between the first one of struts 20 of second subset 34 and at least a second one of struts 20 of first subset 32. The angle is between 5 and 60 degrees, such as between 10 and 30 degrees. For some applications, the first tip portion 50 is disposed axially near (e.g., within 2 mm of) an axial location 52 along stent-graft 10 corresponding with the greatest bulge radius R_{B}, while for other applications, the first tip portion 50 axially crosses bulge 42 and terminates more than 2 mm from axial location 52. As used in the present application, including in the claims, a "tip portion" is a free end of a strut 20 of second subset 34; the free end is disposed radially outward from the substantially cylindrical tubular portion 40 from which the tip portion extends, when stent-graft 10 assumes the radially-expanded state. By "free end" it is meant a free end from other portions of struts 20; the tip portion may be sutured to fluid flow guide 30, as described hereinbelow.

For some applications, the first one of struts 20 of second subset 34 has a length measured along the first one of struts 20 between (a) the first tip portion 50 and (b) a junction between the first one of struts 20 of second subset 34 and the second one of struts 20 of first subset 32. A far half of the first one of struts 20 extends from the first tip portion 50 along 50% of the length of the first one of struts 20 of second subset 34. Stent-graft 10 further comprises sutures that secure struts 20 of first subset 32 and second subset 34 to fluid flow guide 30. None of the sutures are disposed along at least 50% of the far half of the first one of struts 20 of second subset 34. This configuration provides a degree of freedom of motion for the struts of the second subset. This freedom allows the struts of the second subset to better accommodate the disturbance of a branching stent-graft, which may provide better sealing between the bulge and the branching stent-graft. In this configuration, atraumatic features 84, described hereinbelow, typically do not serve as suture rings.

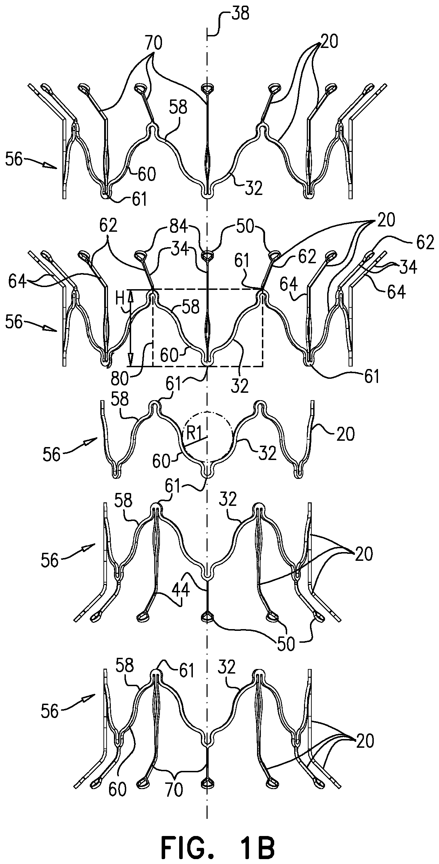

For some applications, as labeled in Fig. 1B, struts 20 of first subset 32 are arranged in a plurality of undulating circumferentially-continuous rings 56 having alternating peaks 58 and troughs 60 (i.e., apices pointing in opposite axial directions). For some applications, peaks 58 and troughs 60 are substantially semicircular, have a first average radius of curvature R1, and are shaped so as to define respective strain-distribution features 61 having a second average radius of curvature equal to between 10% and 50% of the first average radius of curvature. Optionally, strain-distribution features 61 are disposed on proximal-most and/or distal-most points of peaks 58 and/or troughs 60.

For some applications, a first plurality 62 of struts 20 of second subset 34 originate in (i.e., are joined to and extend from) a proximal half of one of undulating circumferentially-continuous rings 56, and a second plurality 64 of struts 20 of second subset 34 originate in a distal half of the one of the undulating circumferentially-continuous rings 56. Typically, struts 20 of the first plurality 62 and the second plurality 64 extend in the same axial direction (e.g., proximally, as shown in the set of struts 20 labeled in Fig. 1B) (although the struts also extend with a radially-outward directional component). For some applications, first plurality 62 of struts 20 of second subset 34 originate in a proximal 20% of an axial height H of the one of the undulating circumferentially-continuous rings 56, such as at proximal-most sites of the one of the undulating circumferentially-continuous rings 56. Alternatively or additionally, for some applications, second plurality 64 of struts 20 of second subset 34 originate in a distal 20% of the axial height H of the one of the undulating circumferentially-continuous rings 56, such as at distal-most sites of the one of the undulating circumferentially-continuous rings 56. For some applications, first plurality 62 of struts 20 of second subset 34 originate at two or more axially different locations of one of the undulating circumferentially-continuous rings 56.

Alternatively, for some applications, at least one of struts 20 of second subset 34 is not structurally integral with any of struts 20 of first subset 32 (configuration not shown).

For some applications, as labeled in Fig. 1A, fluid flow guide 30 is fixed to first subset 32 of struts 20, second subset 34 of struts 20, and a fourth subset 70 of struts 20 so as to define lumen 36; first, second, and fourth subsets 32, 34, and 70 do not include any common struts 20. Struts 20 of fourth subset 70 are elastic such that, when stent-graft 10 assumes the radially-expanded state, struts 20 of fourth subset 70 cause fluid flow guide 30 to define a flared axial portion 72 that extends to one end 74 of fluid flow guide 30, flared axial portion 72 having a greatest flared radius R_{F} from central longitudinal axis 38, which greatest flared radius R_{F} is at least 5% greater than a radius R_{C} of the substantially cylindrical tubular portion 40 axially adjacent flared axial portion 72. Typically, flared axial portion 72 has an axial length L_{F} equal to between 5% and 20% of a difference between (i) the greatest flared radius R_{F} and (ii) the radius R_{C} of the substantially cylindrical tubular portion 40 axially adjacent flared axial portion 72.

For some applications, flared axial portion 72 is a first flared axial portion 72A, and the one end 74 of fluid flow guide 30 is a first end 74A of fluid flow guide 30. Struts 20 of fourth subset 70 are elastic such that, when stent-graft 10 assumes the radially-expanded state, struts 20 of fourth subset 70 cause fluid flow guide 30 to additionally define a second flared axial portion 72B that extends to a second end 74B of fluid flow guide 30, second flared axial portion 72B having a greatest flared radius from central longitudinal axis 38, which greatest flared radius is at least 5% greater than a radius of the substantially cylindrical tubular portion 40 axially adjacent second flared axial portion 72B. Typically, second flared axial portion 72B has an axial length equal to between 5% and 20% of a difference between (i) the greatest flared radius and (ii) the radius of the substantially cylindrical tubular portion 40 axially adjacent second flared axial portion 72B.

Flared axial portions 72 may serve the same function as bulges 42. Providing flared axial portions 72 at the ends of stent-graft 10, rather than bulges 42 at the ends may serve to prevent blood from pooling and circulating in the bulges, which might cause thrombosis.

For some applications, stent-graft 10 further comprises sutures that secure struts 20 of first and second subsets 32 and 34 to fluid flow guide 30, and at least 80% of the surface area of struts 20 of first subset 32 is within 3 mm of at least one of the sutures that secure struts 20 of first subset 32 to fluid flow guide 30. For some applications, no more than 50% of the surface area of struts 20 of second subset 34 is within 3 mm of at least one of the sutures that secure struts 20 of second subset 34 to fluid flow guide 30.

For some applications, such as shown in Fig. 1C, struts 20 of second subset 34 have an average wall thickness T1, measured radially, that is no more than 80% (e.g., no more than 60%) of an average wall thickness T2 of struts 20 of first subset 32, measured radially. As used in the present application, including in the claims, the wall thickness of a strut is "measured radially" by measuring the distance between the surfaces of the strut that are farther and closer to central longitudinal axis 38 when the strut is oriented parallel with central longitudinal axis 38 (even though this is not necessarily its resting, unconstrained orientation). Such a lower wall thickness may contribute to a lower spring constant of struts 20 of second subset 34 than struts 20 of first subset 32, which may facilitate more local deformation (indentation) of bulge 42 around a branching stent-graft, without unnecessarily crushing the branching stent-graft, or necessitating internally reinforcement of the branching stent-graft with additional metallic stents, such as described hereinabove with reference to Figs. 2A-D. For some applications, the lesser wall thickness T1 is achieved by electropolishing struts 20 of second subset 34 for a longer duration than struts 20 of first subset 32, thereby removing more material from those of the second subset. Alternatively or additionally, for some applications, (a) struts 20 of first subset 32 have a first average cross-sectional area, measured perpendicular to respective axes of struts 20 of first subset 32, (b) struts 20 of second subset 34 have a second average cross-sectional area, measured perpendicular to respective axes of struts 20 of second subset 34, and (c) the second average cross-sectional area is no more than 80% of the first cross-sectional area.

Typically, for applications in which stent-graft 10 defines second bulge 42B, such as described hereinabove, struts 20 of third subset 44 have an average wall thickness, measured radially, that is no more than 80% of the average wall thickness of struts 20 of first subset 32, measured radially.

Reference is still made to Figs. 1A-C. Struts 20 of first subset 32 are arranged as a plurality of circumferential cells 80 in circumferentially-continuous rings 56, which cause fluid flow guide 30 to define the plurality of substantially cylindrical tubular portions 40 (for clarity of illustration, a single one of circumferential cells 80 is labeled in Fig. 1B). As shown in Fig. 1B, each of circumferential cells 80 circumferentially begins and ends at a same axial location, i.e., includes exactly one period (cycle) of struts 20 of first subset 32. It is noted that a period (cycle) is to be understood as the smallest possible repeating unit of struts 20, and thus two identical circumferentially adjacent cells define two periods, each having a certain width, rather than a single period having twice the certain width. (Optionally, not all of circumferentially-continuous rings 56 are connected to or associated with struts 20 that define a bulge 42 or a flared axial portion 72.) As mentioned above, struts 20 of second subset 34 define tip portions 50 (which are optionally, but not necessarily, disposed axially near (e.g., within 2 mm of) axial location 52 along stent-graft 10 corresponding with the greatest bulge radius R_{B}). The number of tip portions 50 of struts 20 of second subset 34 that define bulge 42 is at least 50% greater than the average number of circumferential cells 80 in the two circumferentially-continuous rings 56 proximally and distally adjacent bulge 42 (i.e., axially surrounding bulge 42), such as least 175% greater, no more than 250% greater, and/or between 175% and 250% (e.g., 200%) of the average number of circumferential cells 80. For some applications, the number of the tip portions of struts 20 of second subset 34 that define bulge 42 equals at least 12, no more than 24, and/or between 12 and 24. Providing such a relative or absolute large number of tip portions 50 may facilitate more local deformation (indentation) of bulge 42 around a branching stent-graft, without unnecessarily crushing the branching stent-graft, or necessitating internally reinforcement of the branching stent-graft with additional metallic stents, such as described hereinbelow with reference to Figs. 2A-D.

For some applications, each of circumferentially-continuous rings 56 is shaped so as to define between 4 and 16 circumferential cells 80. For some applications, at least 75% of circumferential cells 80 of each circumferentially-continuous ring 56 are similarly shaped. For some applications, the dimensions of circumferential cells 80 of each circumferentially-continuous ring 56 differ between circumferentially adjacent cells 80.

For some applications, tip portions 50 are directly sutured to fluid flow guide 30. For some of these applications, tip portions 50 are shaped so as to define respective atraumatic features 84 (some of which are labeled in Fig. 1B), which optionally, in some configurations, serve as suture rings which are directly sutured to fluid flow guide 30. Optionally, atraumatic features 84 are substantially circular, as shown. For some applications, at least some of tip portions 50 comprise respective radiopaque markers.

For some applications, circumferential cells 80 are W-shaped, V-shaped, U-shaped, or M-shaped (configurations not shown) or diamond-shaped (having a constant or varying width (configuration not shown).

Reference is now made to Figs. 2A-D, which are schematic illustration of exemplary deployments of an endovascular system 100 in an aneurysmal descending aorta 150, in accordance with respective applications of apparatus embodying the present invention. This "periscope" configuration in a "sandwich" assembly is typically used for treating dilations that are distal (i.e., downstream) to branching arteries, such as left and right renal arteries 152A and 152B. In this deployment, stent-graft 10 is a main stent-graft 10. Techniques for deployment may be used that are described in one or more of the patent applications incorporated hereinbelow by reference, or otherwise known in the art. Although the deployment is illustrated with reference to the descending aorta, renal arteries, SMA, and the celiac artery, endovascular system 100 may also be deployed in the vicinity of other main and branching blood vessels, such as arteries, e.g., visceral arteries.

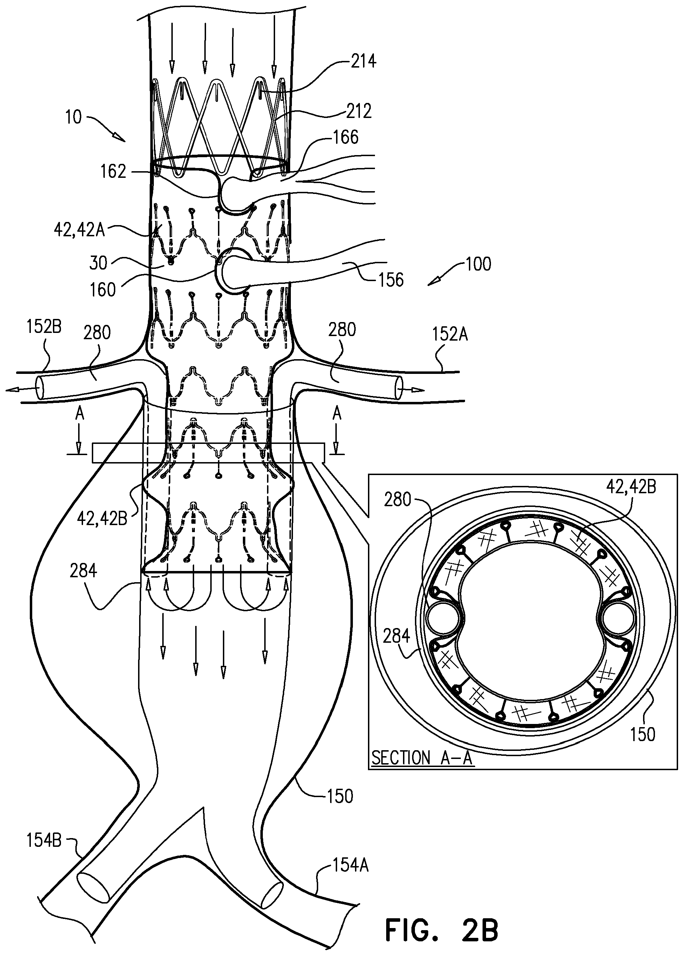

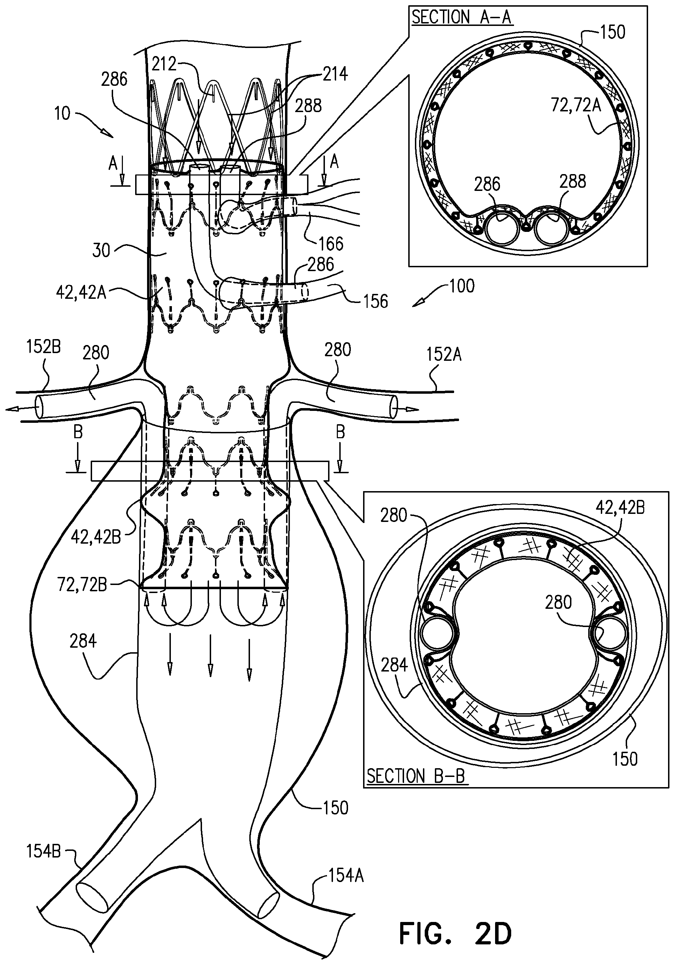

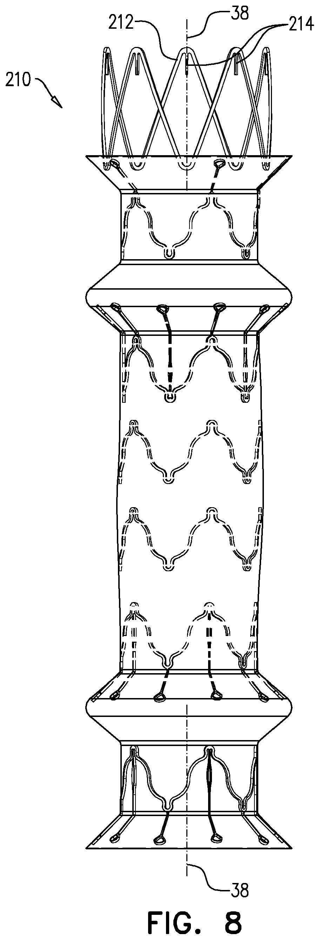

In the configurations shown in Figs. 2B and 2D, stent-graft 10 comprises a plurality of bare struts 212, such as described hereinabove with reference to Fig. 8.

Figs. 2A-D show endovascular system 100 upon deployment of:
- endovascular stent-graft 10 in descending aorta 150, spanning left and right renal arteries 152A and 152B,
- two branching stent-grafts 280, positioned extending (a) side-by-side (i.e., in parallel with) a portion of endovascular stent-graft 10 distal (i.e., downstream) to branching left renal and right renal arteries 152A and 152B, and (b) into the renal arteries; these branching prostheses thus provide a blood-flow path from the main artery to the branching arteries. Second bulge 42B reduces the likelihood of long-term leakage (i.e., blood flow) between "gutters" between endovascular stent-graft 10 and branching stent-grafts 280; as a result, the likelihood of type 1 endoleak is reduced. In addition, the strut-structure of second bulge 42B may facilitate more local deformation (indentation) of the bulge around branching stent-graft 280, without unnecessarily crushing branching stent-grafts 280, or necessitating internally reinforcement of branching stent-grafts 280 with additional metallic stents, and
- at least one extension endovascular stent-graft 284, which bypasses the aneurysmal sac to left and right iliac arteries 154A and 154B, and which is sealingly coupled to endovascular stent-graft 10 during the deployment procedure.

Typically, respective distal ends of branching stent-grafts 280 are disposed at or near a distal end of endovascular stent-graft 10, such as within 2 cm of the distal end of endovascular stent-graft 10 (either proximal or distal the distal end). Respective proximal ends of branching stent-grafts 280 are disposed in left and right renal arteries 152A and 152B.

In the configuration shown in Figs. 2A and 2B, fluid flow guide 30 is shaped so as to define:
- at least one fenestration 160 through fluid flow guide 30. For some applications, a perimeter of fenestration 160 equals between 20% and 80% (e.g., between 20% and 40%) of a perimeter of stent-graft 10 at an axial location of fenestration 160. For some applications, fenestration 160 is substantially circular, such as circular; and/or
- at least one scallop 162. Typically, a circumference of scallop 162 is between 25 and 50 mm. For some applications, scallop 162 is U-shaped, V-shaped, rectangularly-shaped, or semicircularly shaped. For some applications, scallop 162 is disposed proximally (i.e., upstream) to fenestration 160.

In the configuration shown in Figs. 2A and 2B, stent-graft 10 is disposed such that fenestration 160 provides a blood-flow path to superior mesenteric artery (SMA) 156 (which is on the anterior surface of the aorta), and scallop 162 provides a blood-flow path to celiac artery 166. (Thus, endovascular system 100 comprises a number of branching stent-grafts, the number greater than a number of the openings (fenestrations and/or scallops).

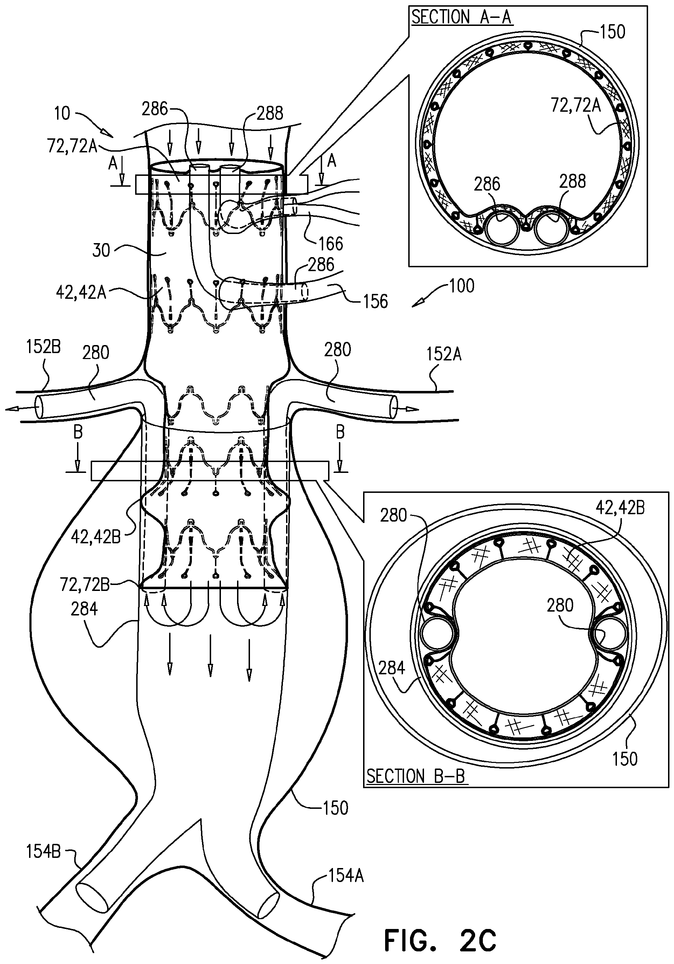

In the configuration shown in Figs. 2C and 2D, fluid flow guide 30 is not shaped so as to define fenestration 160 or scallop 162. Instead, two additional branching stent-grafts 286 and 288 are deployed alongside a proximal portion of stent-graft 10 in a "chimney" configuration. First bulge 42A and/or first flared axial portion 72A prevent leakage from the chimney. In addition, the strut-structure of first bulge 42A may facilitate more local deformation (indentation) of the bulge around branching stent-grafts 286 and 288, without unnecessarily crushing branching stent-grafts 286 or 288, or necessitating internally reinforcement of branching stent-grafts 286 or 288 with additional metallic stents.

In the configurations shown in all of Figs. 2A-D, upon deployment of all of the endoluminal stent-grafts, multi-component endovascular system 100 defines a blood-flow path from upstream of the renal arteries to the renal arteries, SMA, celiac artery, and iliac arteries. A combination of the configurations of Figs. 2A, 2B, 2C, and 2D may be used.

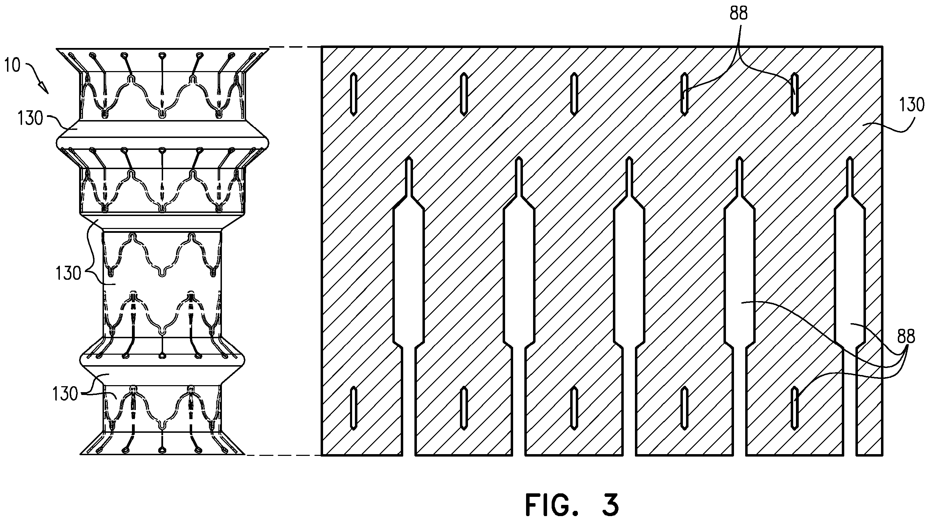

Reference is made to Fig. 3, which is a schematic illustration of cutting pattern for the fabric of fluid flow guide 30 during fabrication. During fabrication of fluid flow guide 30, portions 88 of the fabric of fluid flow guide 30 are cut away, in order to provide fluid flow guide 30 with different diameters therealong corresponding to the differing diameters of stent-graft 10.

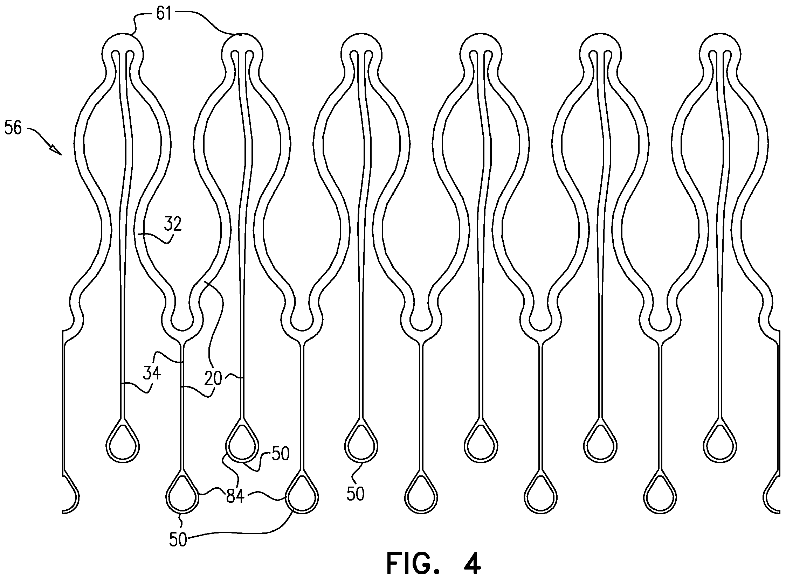

Reference is made to Fig. 4, which is a schematic illustration of a single undulating circumferentially-continuous ring 56. In this view, undulating circumferentially-continuous ring 56 is shown cut at a circumferential site of one of struts 20 of first subset 32 and laid flat.

For some applications, when endovascular stent-graft 10 is removably disposed in the delivery catheter in the radially-compressed state, struts 20 of first subset 32 do not coincide with struts 20 of second subset 34. In other words, if struts 20 are cut at a circumferential site of one of struts 20 of first subset 32 and laid flat, as shown in Fig. 4, struts 20 of first subset 32 do not overlap struts 20 of second subset 34, i.e., do not occupy any of the same locations in the plane. This arrangement of struts 20 avoids the increased crossing profile in the radially-compressed delivery state that would result if the struts overlapped one another.

For some applications, struts 20 of first and second subsets 32 and 34 are fabricated from a single piece of a tubular material (e.g., by laser cutting the material). For some applications, at least one of struts 20 of second subset 34 is structurally integral with at least one of struts 20 of first subset 32, such as shown in the figures. For some applications, all of struts 20 of second subset 34 are structurally integral with at least one of struts 20 of first subset 32, none of struts 20 of second subset 34 is directly connected to any of the other struts of second subset 34, and none of struts 20 of second subset 34 is indirectly connected to any of the other struts of second subset 34 by any struts other than struts 20 of first subset 34. (Struts 20 of second subset 34 are typically indirectly connected by the fluid flow guide 30.) This arrangement of struts 20 allows each circumferential portion of bulge 42 to radially expand generally separately from one another, because circumferentially-adjacent end portions of struts 20 of second subset 34 do not pull on each other.

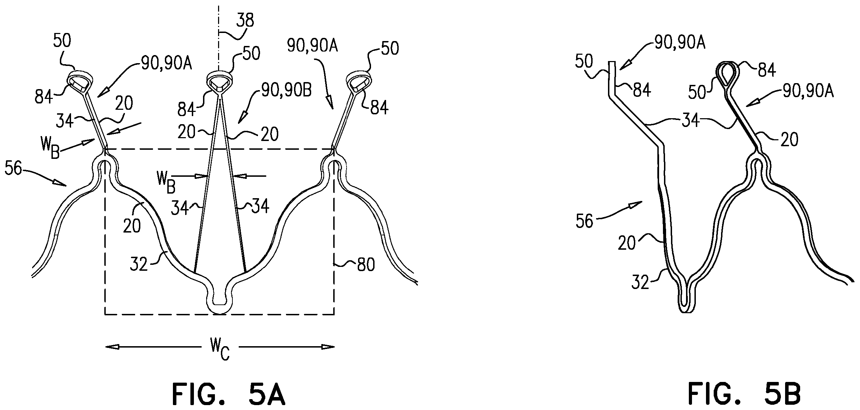

Reference is made to Figs. 5A-B, which are schematic illustrations of alternative configurations of a single undulating circumferentially-continuous ring 56. Only a portion of undulating circumferentially-continuous ring 56 is shown in Figs. 5A-B. These configurations may be used with any of the configuration of stent-graft 10 described herein, *mutatis mutandis.*

In these configurations, struts 20 of second subset 34 are arranged as a plurality of bulge-inducing units 90 that cause fluid flow guide 30 to define bulge 42. Typically, an average circumferential width W_{B} of bulge-inducing units 90 is no more than 25% (e.g., no more than 20%, no more than 15%, or no more than 10%) of an average circumferential width W_{C} of circumferential cells 80 in the two circumferentially-continuous rings 56 proximally and distally adjacent bulge 42 (i.e., axially surrounding bulge 42) (only one of these two circumferentially-continuous rings 56 is shown in Figs. 5A and 5B). The narrow widths of bulge-inducing units 90 may facilitate more local deformation (indentation) of bulge 42 around a branching-stent graft, without unnecessarily crushing the branching stent-graft, or necessitating internally reinforcement of the branching stent-graft with additional metallic stents, such as described hereinabove with reference to Figs. 2A-D. (The "circumferential width" is measured around the circumference of undulating circumferentially-continuous ring 56, as though the ring were cut and laid flat.) For some applications, at least one of (e.g., all of) bulge-inducing units 90 has an aspect ratio (length/width) of at least 4.

For some applications, such as shown in Fig. 5A, all or a portion of bulge-inducing units 90 comprise two struts 20, as shown for bulge-inducing unit 90B, and/or all or a portion of bulge-inducing units 90 comprise exactly one strut 20, which is connected to first subset 32 at exactly one junction, as shown for bulge-inducing units 90A. (The average circumferential width W_{B} of bulge-inducing units 90B equals the weighted average circumferential width of tip portion 50 and the exactly one strut 20 of second subset 34.) Fig. 5A shows a configuration in which bulge-inducing units 90A alternate with bulge-inducing units 90A, while Fig. 5B shows a configuration with only bulge-inducing unit 90A. Thus, in some other configurations described herein (e.g., as shown in Figs. 1A-C, 4, 6, 7, 8, and 9A-B), struts 20 of second subset 34 are arranged as a plurality of bulge-inducing units 90, each of which comprises exactly one strut 20 connected to first subset 32 at exactly one junction.

For some applications, the number of bulge-inducing units 90 is at least 30% (typically at least 50%) greater than the average number of circumferential cells 80 in the two circumferentially-continuous rings 56 proximally and distally adjacent bulge 42 (i.e., axially surrounding bulge 42), such as least 175% greater, no more than 250% greater, and/or between 175% and 250% (e.g., 200%) of the average number of circumferential cells 80.

For some applications, each of bulge-inducing units 90 circumscribes an arc having an angle of between 3 and 20 degrees, e.g., between 5 and 15 degrees.

As described hereinabove with reference to Figs. 1A-C, at least one of struts 20 of second subset 34 defines a tip portion 50. Tip portions 50 may be shaped so as to define respective atraumatic features 84, which are optionally substantially circular. For some applications, such as shown in Fig. 5B, atraumatic features 84 of tip portions 50 are oriented generally parallel to a cylindrical surface defined by first subset 32 of struts 20. This orientation may reduce the risk of damage to fluid flow guide 30 by tip portions 50 in bulges 42.

Reference is again made to Figs. 1A-C. For some applications, a circumference of a first one of substantially cylindrical tubular portions 40 (e.g., 40A or 40B) is at least 10% greater than a circumference of a second one of substantially cylindrical tubular portions 40 (e.g., 40C or 40D).

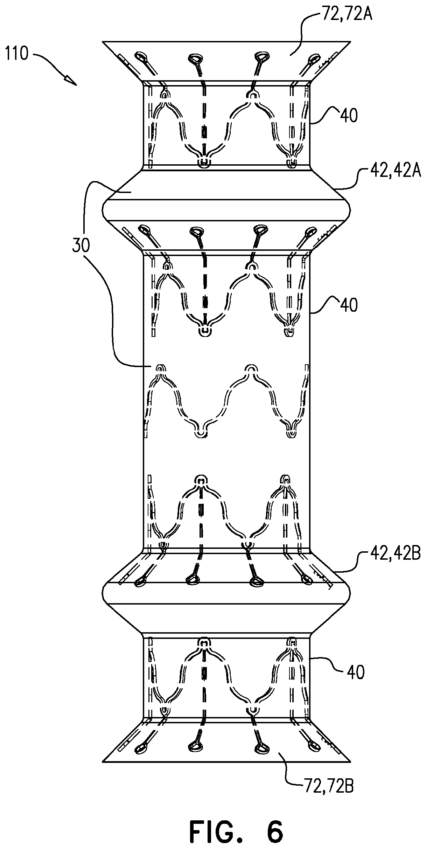

Reference is made to Fig. 6, which is a schematic illustration of an endovascular stent-graft 110, in accordance with an application of the present invention. Except as described below, endovascular stent-graft 110 is similar to endovascular stent-graft 10, described hereinabove with reference to Figs. 1A-5B, and may implement any of the features thereof, *mutatis mutandis.* In endovascular stent-graft 110, respective circumferences of all substantially cylindrical tubular portions 40 of fluid flow guide 30 vary by less than 10%, such as are equal.

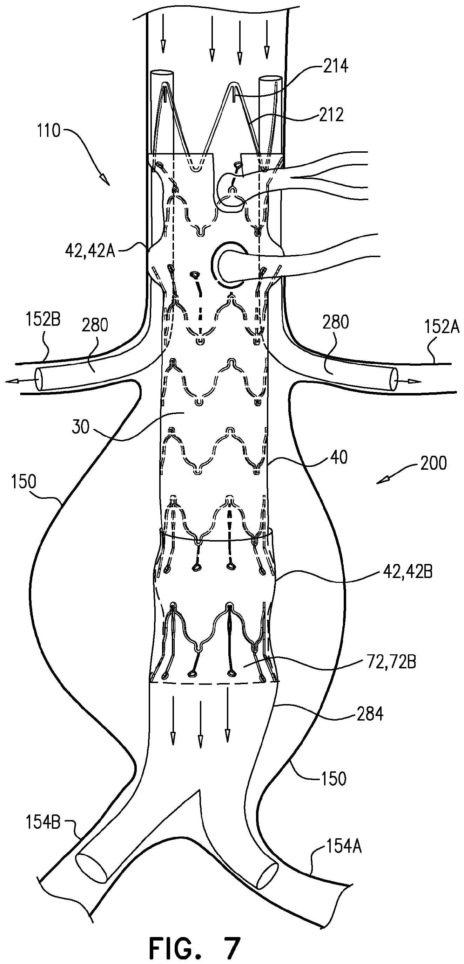

Reference is now made to Fig. 7, which is a schematic illustration of an exemplary deployment of an endovascular system 200 in aneurysmal descending aorta 150, in accordance with an application of apparatus embodying the present invention. This "periscope" configuration in a "sandwich" assembly is typically used for treating dilations that are distal (i.e., downstream) to branching arteries, such as left and right renal arteries 152A and 152B. In this deployment, stent-graft 110 is a main stent-graft 110. Techniques for deployment may be used that are described in one or more of the patent applications incorporated hereinbelow by reference, or otherwise known in the art.

Fig. 7 shows endovascular system 200 upon deployment of:
- endovascular stent-graft 110 in descending aorta 150, spanning left and right renal arteries 152A and 152B, after delivery catheter 772 has been withdrawn to release stent-graft 10, and
- two branching stent-grafts 280, positioned partially extending along a proximal portion of endovascular stent-graft 110 in a "chimney" configuration and into respective branching arteries: left renal artery 152A and right renal artery 152B; first bulge 42A and/or first flared axial portion 72A reduce the likelihood of long-term leakage (i.e., blood flow) through "gutters." As a result, the likelihood of type 1 endoleak is reduced.

Typically, respective proximal ends of branching stent-grafts 280 are disposed at or near a proximal end of endovascular stent-graft 110, such as within 2 cm of the proximal end of endovascular stent-graft 110 (either proximal or distal the proximal end). Preferably, the respective proximal ends of branching stent-grafts 280 are disposed not proximally to the proximal end of endovascular stent-graft 110, because if they were disposed proximally to the proximal end of endovascular stent-graft 110, blood flow might cause them to bend, curve, and whip in accordance with the aortic systole cycle. Respective distal ends of branching stent-grafts 280 are disposed in left and right renal arteries 152A and 152B.

Optionally, endovascular system 200 includes one or more extension endovascular stent-grafts 284, which collectively bypass the aneurysmal sac to left and right iliac arteries 154A and 154B. The extension endovascular prostheses are sealingly coupled to endovascular stent-graft 110 during the deployment procedure. For some applications, main stent-graft 110, other than at bulges 42, is oversized about 15% compared to extension endovascular stent-grafts 284. As can be seen in Fig. 7, upon deployment of all of the endoluminal prostheses, multi-component endovascular system 200 defines a blood-flow path from upstream of the renal arteries to the renal arteries, SMA, celiac artery, and iliac arteries. For some applications, a distal portion of extension endovascular stent-graft 284 is bifurcated, as shown in Fig. 7.

Reference is made to Fig. 8, which is a schematic illustration of an endovascular stent-graft 210, in accordance with an application of the present invention. Except as described below, endovascular stent-graft 210 is similar to endovascular stent-grafts 10 and 110, described hereinabove with reference to Figs. 1A-5B and 6-7, respectively, and may implement any of the features thereof, *mutatis mutandis.* Endovascular stent-graft 210 comprises a plurality of bare struts 212 at a proximal end of the stent-graft. Optionally, one or more of bare struts 212 are shaped so as define fixation barbs 214, which may be oriented at an angle of at least 20 degrees with central longitudinal axis 38. For some applications, at least 10% (e.g., at least 50%) of an axial length of stent-graft 210 is not covered by fluid flow guide 30 (i.e., has only bare struts 212.

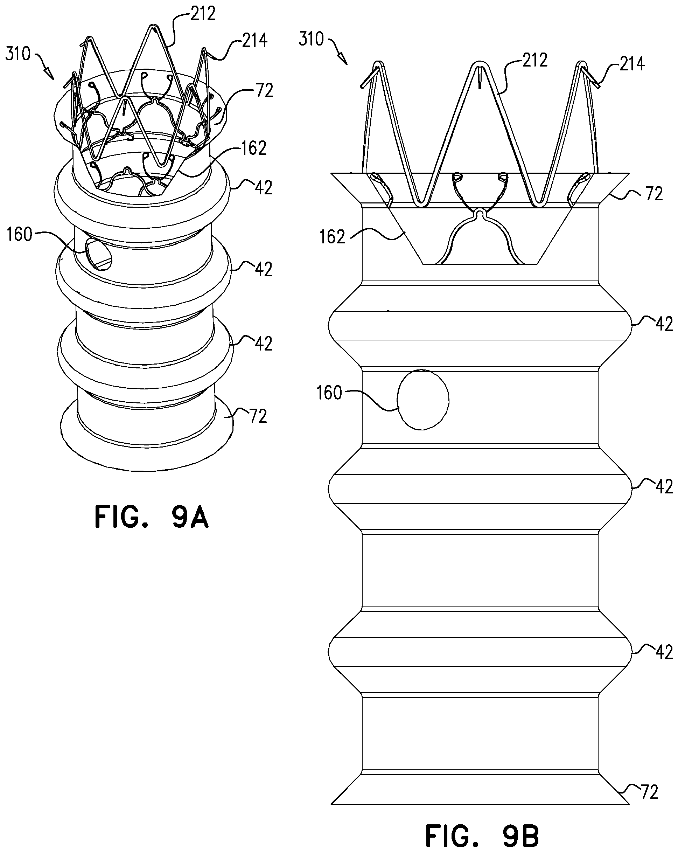

Reference is made to Figs. 9A-B, which are schematic illustrations of an endovascular stent-graft 310, in accordance with an application of the present invention. Except as described below, endovascular stent-graft 310 is similar to endovascular stent-grafts 10, 110, and 210 described hereinabove with reference to Figs. 1A-5B, 6-7, and 8, respectively, and may implement any of the features thereof, *mutatis mutandis.*

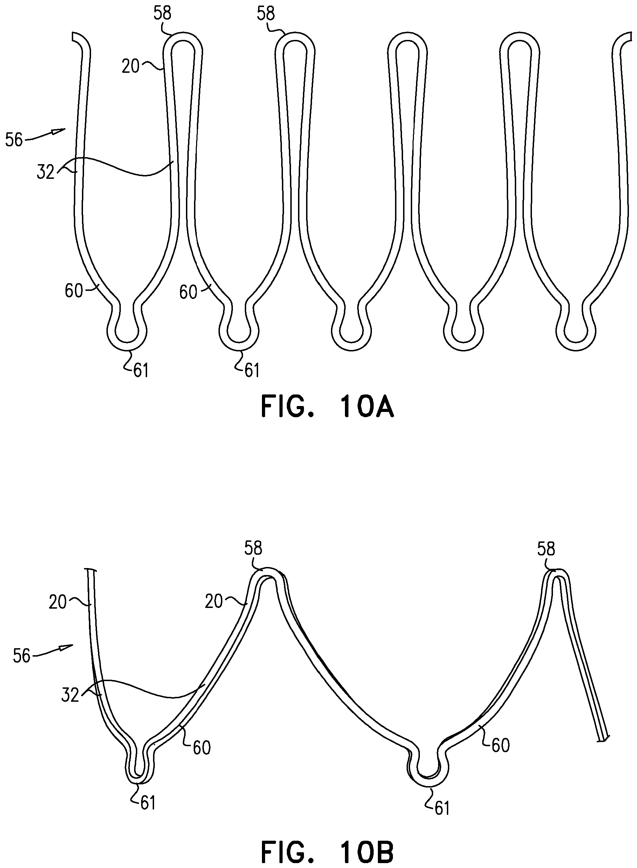

Reference is made to Figs. 10A-B, which are schematic illustrations of a single undulating circumferentially-continuous ring 56 when stent-graft 10 assumes the radially-compressed delivery state and the radially-expanded state, respectively, in accordance with an application of the present invention. Figs. 10A-B show only first subset 32 of struts 20, and do not show second subset 34 of struts 20, which are provided, such as shown, for example, in Figs. 1A-B, 4, 5A-B, and/or 11.

In the configuration shown in Figs. 10A-B, unlike in the configuration shown, for example, in Figs. 1A-B, only troughs 60 are shaped so as to define respective strain-distribution features 61. For some applications, peaks 58, and struts 20 of first subset 32 between peaks 58, together define a convex bell shape having a relatively large opening that can accommodate the aorta disturbance, by directly the disturbance into the opening. Alternatively, only peaks 58 are shaped so as to define respective strain-distribution features 61 (configuration not shown).

For some applications, as shown in Figs. 1A-C, said array of elongated members is structurally integrated with struts 20 of first subset 32 via yet another substantially elongated member. For some applications, as shown in Figs. 1A-C, an end of elongated member has a radius of curvature that is at least 150% of the lowest width of said member.

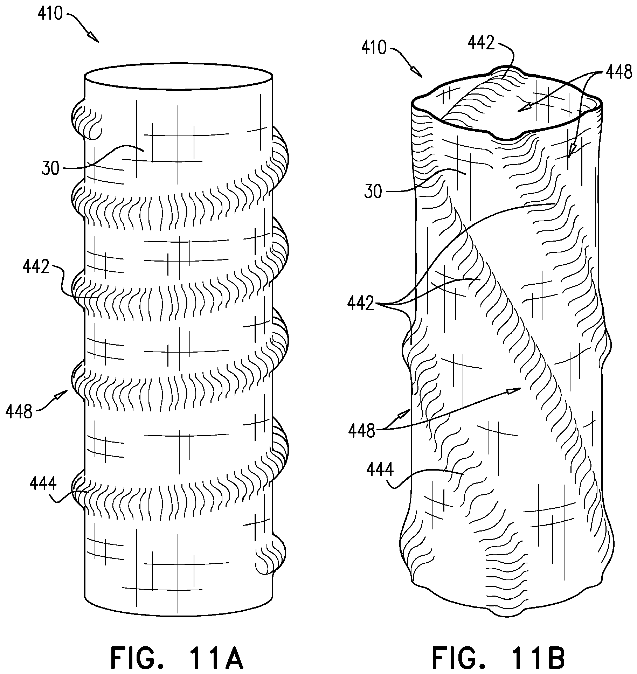

Reference is made to Figs. 11A-B, which are schematic illustrations of an endovascular stent-graft 410. Except as described below, endovascular stent-graft 410 is similar to endovascular stent-grafts 10, 110, 210, and 310 described hereinabove with reference to Figs. 1A-5B, 6-7, 8, and 9A-B, respectively, and may implement any of the features thereof, *mutatis mutandis.* Endovascular stent-graft 410 comprises one or more bulges 442, which bulge radially outward (i.e., away from a central longitudinal axis of fluid flow guide 30), and are arranged as one or more respective circumferential helices 448, each of which circumscribes at least 0.3 complete turns, such as at least 0.5 complete turns, e.g., at least 1.5, 2, or 3 complete turns, around endovascular stent-graft 410.

For some applications, such as shown in Fig. 11A, endovascular stent-graft 410 comprises exactly one bulge 442.

For other applications, such as shown in Fig. 11B, endovascular stent-graft 410 comprises a plurality of bulges 442, e.g., exactly two bulges 442, exactly three bulges 442, exactly four bulges 442 (as shown in Fig. 11B), or five or more bulges 442. For example, the plurality of helices 448 may be arranged as a n-tuple helix, e.g., a double helix, a triple helix, or a quadruple helix (as shown in Fig. 11B). As used in the present application, including in the claims, an "n-tuple helix" comprises n helices with the same axis, differing by a translation along the axis.

For some applications, the one or more bulges 442 are configured to reduce the likelihood of long-term leakage (i.e., blood flow) through gutters, i.e., the residual intravascular space disposed outside the lumens of endovascular stent-graft 410 and branching stent-grafts disposed alongside endovascular stent-graft 410. Because the one or more bulges 442 are arranged as respective helices 448, the one or more bulges 442 contact the branching stent-grafts regardless of the circumferential location of the branching stent-grafts around endovascular stent-graft 410.

Alternatively or additionally, the one or more bulges 442 may reduce the likelihood of blood flow between endovascular stent-graft 410 and features of the anatomy of the blood vessel wall, such as isolated regions of plaque, calcifications, or thrombus, all of which alter the circularity of the blood vessel wall and might otherwise present issues for good sealing between stent-graft 410 and the blood vessel wall. As a result, the likelihood of type 1 endoleak is reduced.

For some application, such as shown in Fig. 11A, the pitch of the one or more helices 448 is less than an axial length of an axial portion of endovascular stent-graft 410 that comprises the one or more bulges 442, e.g., less than 50% of the axial length, e.g., less than 33% of the axial length. For other applications, such as shown in Fig. 11B, the pitch of the one or more helices 448 is greater than the axial length of the axial portion of endovascular stent-graft 410 that comprises the one or more bulges 442, e.g., greater than 150% of the axial length, e.g., greater than 200% of the axial length.

For some applications, such as shown in Fig. 11A, each of the one or more bulges 442 comprises at least one biologically-compatible substantially blood-impervious fabric 444. Fabric 444 is distinct from the fabric of fluid flow guide 30; for example, an inner surface of the fabric of fluid flow guide 30 defines a blood-flow path through fluid flow guide 30, while fabric 444 does not. The one or more bulges 442 are coupled to an external surface of fluid flow guide 30. Typically, each of the one or more bulges 442 further comprises one or more springs, which provide structure to the one or more bulges 442, and are disposed between fabric 444 of the one or more bulges and the fabric of fluid flow guide 30. For example, the one or more springs may comprise helical springs, and fabric 444 may surrounds a portion of the springs, such that the springs are disposed between fabric 444 and fluid flow guide 30. Typically, fabric 444 is sutured to fluid flow guide 30.

For other applications, such as shown in Fig. 11B, the one or more bulges 442 are defined by the fabric of fluid flow guide 30 and the same struts that define lumen 36 of fluid flow guide 30.

It is noted that the features of this configuration shown in Fig. 11B may be implemented in combination with the features shown in Fig. 11A, and *vice versa.*

Typically, each of the one or more bulges 442 has a greatest bulge radius from a central longitudinal axis of fluid flow guide 30 (labeled in Fig. 1C for endovascular stent-graft 10), which greatest bulge radius is at least 5% greater (e.g., at least 10% greater, such as at least 15% greater) than an average radius of substantially cylindrical tubular portions of endovascular stent-graft 410 (i.e., without including bulged portions of the endovascular stent-graft in the calculation of the average radius).

For some applications, the one or more bulges 442 extend to one or both ends of endovascular stent-graft 410, such as shown in Fig. 11B, while for other applications, the one or more bulges 442 do not extend to one or both ends of endovascular stent-graft 410, such as shown in Fig. 11A.

For some applications, the apparatus is substantially tubular on a first end portion and bifurcates to two substantially tubular sub-lumens on its second end portion, creating a continuous space between first end portion and sub-lumens on its second end portion. For some applications, an average circumference of each of its sub-lumens is between 40% and 70% of an average circumference of its first end portion.

Reference is again made to Fig. 3. For some applications, struts 20 of first subset 32 define a conical angle with respect to axis of said stent-graft, said conical angle between 10 to 30 degrees with respect to the angle of the lumen, when the apparatus is in its radially-expanded state.

In an embodiment, techniques and apparatus described in one or more of the following patents and patent applications, which are assigned to the assignee of the present application, are combined with techniques and apparatus described herein.
- PCT Application PCT/IL2008/000287, filed March 5, 2008, which published as PCT Publication WO 2008/107885
- PCT Application PCT/IB2010/052861, filed June 23, 2010, which published as PCT Publication WO 2010/150208
- PCT Application PCT/IL2010/000564, filed July 14, 2010, which published as PCT Publication WO 2011/007354
- PCT Application PCT/IL2010/000917, filed November 4, 2010, which published as PCT Publication WO 2011/055364
- PCT Application PCT/IL2010/000999, filed November 30, 2010, which published as PCT Publication WO 2011/064782
- PCT Application PCT/IL2010/001018, filed December 2, 2010, which published as PCT Publication WO 2011/067764
- PCT Application PCT/IL2010/001037, filed December 8, 2010, which published as PCT Publication WO 2011/070576
- PCT Application PCT/IL2011/000135, filed February 8, 2011, which published as PCT Publication WO 2011/095979
- PCT Application PCT/IL2012/000060, filed February 2, 2012, which published as PCT Publication WO 2012/104842
- PCT Application PCT/IL2012/000241, filed June 19, 2012, which published as PCT Publication WO 2012/176187
- PCT Application PCT/IL2012/000300, filed August 12, 2012, which published as PCT Publication WO 2013/030819
- US Patent 8,317,856 to Shalev et al.
- US Patent 8,574,287 to Benary et al.
- US Provisional Application 60/892,885, filed March 5, 2007
- US Provisional Application 60/991,726, filed December 2, 2007
- US Provisional Application 61/219,758, filed June 23, 2009
- US Provisional Application 61/221,074, filed June 28, 2009
- US Application 13/031,871, filed February 22, 2011, which published as US Patent Application Publication 2011/0208289
- US Provisional Application 61/496,613, filed June 14, 2011
- US Provisional Application 61/499,195, filed June 21, 2011
- US Provisional Application 61/505,132, filed July 7, 2011
- US Provisional Application 61/529,931, filed September 1, 2011
- US Provisional Application 61/553,209, filed October 30, 2011
- US Patent 8,870,938 to Shalev et al.
- US Application 13/384,075, filed January 13, 2012, which published as US Patent Application Publication 2012/0179236
- US Application 13/505,996, filed May 3, 2012, which published as US Patent Application Publication 2012/0310324
- US Application 13/513,397, filed June 1, 2012, which published as US Patent Application Publication 2012/0330399
- US Application 13/514,240, filed June 6, 2012, which published as US Patent Application Publication 2013/0013051
- US Provisional Application 61/678,182, filed August 1, 2012
- US Patent 9,468,517 to Shalev
- US Patent 8,945,203 to Shalev et al.
- US Application 13/807,880, filed December 31, 2012, which published as US Patent Application Publication 2013/0131783
- PCT Application PCT/IL2012/000095, filed March 1, 2012, which published as PCT Publication WO 2012/117395
- PCT Application PCT/IL2012/000148, filed April 4, 2012, which published as PCT Publication WO 2013/030818
- PCT Application PCT/IL2012/000190, filed May 15, 2012, which published as PCT Publication WO 2013/171730
- PCT Application PCT/IL2012/000269, filed July 2, 2012, which published as PCT Publication WO 2013/005207
- PCT Application PCT/IL2012/050424, filed October 29, 2012, which published as PCT Publication WO 2013/065040
- PCT Application PCT/IL2012/050506, filed December 4, 2012, which published as PCT Publication WO 2013/084235
- US Provisional Application 61/749,965, filed January 8, 2013
- US Patent 8,951,298 to Shalev
- US Provisional Application 61/775,964, filed March 11, 2013
- US Provisional Application 61/826,544, filed May 23, 2013
- US Patent 9,855,046 to Shalev
- PCT Application PCT/IL2013/050656, filed July 31, 2013, which published as PCT Publication WO 2014/020609
- US Provisional Application 61/906,014, filed November 19, 2013
- PCT Application PCT/IL2014/050019, filed January 7, 2014, which published as PCT Publication WO 2014/108895
- US Provisional Application 61/926,533, filed January 13, 2014
- PCT Application PCT/IL2014/050174, filed February 18, 2014, which published as PCT Publication WO 2014/141232
- PCT Application PCT/IL2014/050434, filed May 18, 2014, which published as PCT Publication WO 2014/188412
- PCT Application PCT/IL2014/050973, filed November 6, 2014, which published as PCT Publication WO 2015/075708
- US Provisional Application 62/093,497, filed December 18, 2014
- US Provisional Application 62/102,265, filed January 12, 2015
- US Application 14/416,236, filed January 21, 2015, which published as US Patent Application Publication 2015/0202065
- US Provisional Application 62/110,659, filed February 2, 2015
- US Provisional Application 62/254,432, filed November 12, 2015
- PCT Application PCT/IL2015/051221, filed December 16, 2015, which published as PCT Publication WO 2016/098113
- PCT Application PCT/IL2016/050014, filed January 6, 2016, which published as PCT Publication WO 2016/113731
- PCT Application PCT/IL2016/050049, filed January 14, 2016, which published as PCT Publication WO 2016/125137
- US Provisional Application 62/371,983, filed August 8, 2016
- PCT Application PCT/IL2016/051207, filed November 9, 2016, which published as PCT Publication WO 2017/081679

It will be appreciated by persons skilled in the art that the present invention is not limited to what has been particularly shown and described hereinabove. Rather, the scope of the present invention is defined by the claims.

## Claims

1. Apparatus for use with a delivery catheter, the apparatus comprising an endovascular stent-graft (10, 110, 210, 310), which is configured to initially be positioned in the delivery catheter in a radially-compressed state, and to assume a radially-expanded state upon being deployed from the delivery catheter, and which comprises:
struts (20); and
a fluid flow guide (30), which comprises at least one biologically-compatible substantially blood-impervious fabric, and which is fixed to first and second subsets (32, 34) of the struts (20), wherein the first and the second subsets (32, 34) do not include any common struts (20),
wherein the struts (20) of the first and the second subsets (32, 34) are elastic such that, when the stent-graft (10, 110, 210, 310) assumes the radially-expanded state:
the fluid flow guide (30) defines a lumen (36) having a central longitudinal axis (38),
the struts (20) of the first subset (32) are arranged as a plurality of circumferential cells (80) in circumferentially-continuous rings (56), which cause the fluid flow guide (30) to define a plurality of substantially cylindrical tubular portions (40), and
the struts (20) of the second subset (34) cause the fluid flow guide (30) to define a bulge (42) having a greatest bulge radius from the central longitudinal axis (38), which greatest bulge radius is at least 5% greater than an average radius of the substantially cylindrical tubular portions (40) proximally and distally adjacent the bulge (42), **characterised in that**:
the struts (20) of the second subset (34) define tip portions (50), and
the number of the tip portions (50) of the struts (20) of the second subset (34) that define the bulge (42) is at least 50% greater than the average number of circumferential cells (80) in the two circumferentially-continuous rings (56) proximally and distally adjacent the bulge (42).

2. The apparatus according to claim 1, wherein the number of the tip portions (50) of the struts (20) of the second subset (34) that define the bulge (42) equals between 175% and 250% of the average number of circumferential cells (80).

3. The apparatus according to claim 2, wherein the number of the tip portions (50) of the struts (20) of the second subset (34) that define the bulge (42) equals 200% of the average number of circumferential cells (80).

4. The apparatus according to claim 1, wherein the number of the tip portions (50) of the struts (20) of the second subset (34) that define the bulge (42) equals between 12 and 16.

5. The apparatus according to claim 1, wherein the bulge (42) completely circumferentially encircles the stent-graft (10, 110, 210, 310).

6. The apparatus according to claim 1, wherein the tip portions (50) are directly sutured to the fluid flow guide (30).

7. The apparatus according to claim 1, wherein the tip portions (50) are shaped so as to define respective atraumatic features (84).

8. The apparatus according to claim 1, the fluid flow guide (30) is arranged such that all of the fabric defines the lumen (36) when the stent-graft (10, 110, 210, 310) assumes the radially-expanded state.

9. The apparatus according to claim 1,
wherein the circumferentially-continuous rings (56) undulate, such that the circumferential cells (80) are shaped so as to define respective peaks (58) and troughs (60), and
wherein the peaks (58) and the troughs (60) are substantially semicircular, have a first average radius of curvature, and are shaped so as to define respective strain-distribution features (61) having a second average radius of curvature equal to between 10% and 50% of the first average radius of curvature.

10. The apparatus according to any one of claims 1-9,
wherein the bulge (42) is a first bulge (42A),
wherein the fluid flow guide (30) is fixed to the first subset (32) of the struts (20), the second subset (34) of the struts (20), and a third subset (44) of the struts (20) so as to define the lumen (36), wherein the first, the second, and the third subsets (32, 34, 44) do not include any common struts (20), and
wherein the struts (20) of the third subset (44) are elastic such that, when the stent-graft (10, 110, 210, 310) assumes the radially-expanded state:
the struts (20) of the third subset (44) cause the fluid flow guide (30) to define a second bulge (42B) having a greatest bulge radius from the central longitudinal axis (38), which greatest bulge radius is at least 5% greater than an average radius of the substantially cylindrical tubular portions (40) proximally and distally adjacent the second bulge (42B),
the struts (20) of the third subset (44) define tip portions (50), and
the number of the tip portions (50) of the struts (20) of the third subset (44) that define the second bulge (42B) is at least 50% greater than the average number of circumferential cells (80) in the two circumferentially-continuous rings (56) proximally and distally adjacent the second bulge (42B).

11. The apparatus according to any one of claims 1-9, wherein at least a first one of the struts (20) of the second subset (34) is structurally integral with at least a second one of the struts (20) of the first subset (32).

12. The apparatus according to claim 11,
wherein all of the struts (20) of the second subset (34) are structurally integral with at least one of the struts (20) of the first subset (32),
wherein none of the struts (20) of the second subset (34) is directly connected to any of the other struts (20) of the second subset (34), and
wherein none of the struts (20) of the second subset (34) is indirectly connected to any of the other struts (20) of the second subset (34) by any struts (20) other than the struts (20) of the first subset (32).

13. The apparatus according to any one of claims 1-9,
wherein the struts (20) of the first subset (32) are arranged in a plurality of undulating circumferentially-continuous rings (56) having alternating peaks (58) and troughs (60), and
wherein a first plurality (62) of the struts (20) of the second subset (34) originate in a proximal half of one of the undulating circumferentially-continuous rings (56), and a second plurality (64) of the struts (20) of the second subset (34) originate in a distal half of the one of the undulating circumferentially-continuous rings (56).

14. The apparatus according to claim 13,
wherein the first plurality (62) of the struts (20) of the second subset (34) originate at proximal-most sites of the one of the undulating circumferentially-continuous rings (56), and
wherein the second plurality (64) of the struts (20) of the second subset (34) originate at distal-most sites of the one of the undulating circumferentially-continuous rings (56).

15. The apparatus according to any one of claims 1-9,
wherein the fluid flow guide (30) is fixed to the first subset (32) of the struts (20), the second subset (34) of the struts (20), and a third subset (70) of the struts (20) so as to define the lumen (36), wherein the first, the second, and the third subsets (32, 34, 70) do not include any common struts (20), and
wherein the struts (20) of the third subset (70) are elastic such that, when the stent-graft (10, 110, 210, 310) assumes the radially-expanded state, the struts (20) of the third subset (70) cause the fluid flow guide (30) to define a flared axial portion (72) that extends to one end (74) of the fluid flow guide (30), the flared axial portion (72) having (a) a greatest flared radius from the central longitudinal axis (38), which greatest flared radius is at least 5% greater than a radius of the substantially cylindrical tubular portion (40) axially adjacent the flared axial portion (72), and (b) an axial length equal to between 5% and 20% of a difference between (i) the greatest flared radius and (ii) the radius of the substantially cylindrical tubular portion (40) axially adjacent the flared axial portion (72).

16. The apparatus according to any one of claims 1-9, wherein when the endovascular stent-graft (10, 110, 210, 310) is removably disposed in the delivery catheter in the radially-compressed state, the struts (20) of the first subset (32) do not coincide with the struts (20) of the second subset (34).

## Patentansprüche

1. Vorrichtung zur Verwendung mit einem Abgabekatheter, wobei die Vorrichtung ein endovaskuläres Stentgraft (10, 110, 210, 310) umfasst, das dazu konfiguriert ist, anfänglich in einem radial komprimierten Zustand in dem Abgabekatheter positioniert zu sein und einen radial ausgedehnten Zustand nach dem Entfalten aus dem Abgabekatheter anzunehmen, und Folgendes umfasst:
Streben (20); und
eine Fluidströmungsführung (30), die mindestens ein biologisch verträgliches, im Wesentlichen blutundurchlässiges Gewebe umfasst und die an einer ersten und einer zweiten Teilmenge (32, 34) der Streben (20) befestigt ist, wobei die erste und die zweite Teilmenge (32, 34) keine gemeinsamen Streben (20) enthalten,
wobei die Streben (20) der ersten und der zweiten Teilmenge (32, 34) elastisch sind, sodass, wenn das Stentgraft (10, 110, 210, 310) den radial ausgedehnten Zustand annimmt:
die Fluidströmungsführung (30) ein Lumen (36), das eine Mittellängsachse (38) aufweist, definiert,
die Streben (20) der ersten Teilmenge (32) als eine Vielzahl von Umfangszellen (80) in in Umfangsrichtung durchgehenden Ringen (56) angeordnet sind, die bewirken, dass die Fluidströmungsführung (30) eine Vielzahl von im Wesentlichen zylindrischen röhrenförmigen Abschnitten definiert (40), und
die Streben (20) der zweiten Teilmenge (34) bewirken, dass die Fluidströmungsführung (30) eine Ausbuchtung (42), die einen größten Ausbuchtungsradius von der Mittellängsachse (38) aufweist, definiert, wobei der größte Ausbuchtungsradius mindestens 5 % größer als ein durchschnittlicher Radius der im Wesentlichen zylindrischen röhrenförmigen Abschnitte (40) proximal und distal angrenzend an die Ausbuchtung (42) ist, **dadurch gekennzeichnet, dass**:
die Streben (20) der zweiten Teilmenge (34) Spitzenabschnitte (50) definieren, und
die Anzahl der Spitzenabschnitte (50) der Streben (20) der zweiten Teilmenge (34), die die Ausbuchtung (42) definieren, mindestens 50 % größer als die durchschnittliche Anzahl von Umfangszellen (80) in den zwei in Umfangsrichtung durchgehenden Ringen (56) proximal und distal angrenzend an die Ausbuchtung (42) ist.

2. Vorrichtung nach Anspruch 1, wobei die Anzahl der Spitzenabschnitte (50) der Streben (20) der zweiten Teilmenge (34), die die Ausbuchtung (42) definieren, gleich zwischen 175 % und 250 % der durchschnittlichen Anzahl von Umfangszellen (80) ist.

3. Vorrichtung nach Anspruch 2, wobei die Anzahl der Spitzenabschnitte (50) der Streben (20) der zweiten Teilmenge (34), die die Ausbuchtung (42) definieren, gleich 200 % der durchschnittlichen Anzahl von Umfangszellen (80) ist.

4. Vorrichtung nach Anspruch 1, wobei die Anzahl der Spitzenabschnitte (50) der Streben (20) der zweiten Teilmenge (34), die die Ausbuchtung (42) definieren, gleich zwischen 12 und 16 ist.

5. Vorrichtung nach Anspruch 1, wobei die Ausbuchtung (42) das Stentgraft (10, 110, 210, 310) vollständig in Umfangsrichtung umgibt.

6. Vorrichtung nach Anspruch 1, wobei die Spitzenabschnitte (50) direkt an die Fluidströmungsführung (30) genäht sind.

7. Vorrichtung nach Anspruch 1, wobei die Spitzenabschnitte (50) so geformt sind, dass sie jeweilige atraumatische Merkmale (84) definieren.

8. Vorrichtung nach Anspruch 1, wobei die Fluidströmungsführung (30) so angeordnet ist, dass das gesamte Gewebe das Lumen (36) definiert, wenn das Stentgraft (10, 110, 210, 310) den radial ausgedehnten Zustand annimmt.

9. Vorrichtung nach Anspruch 1,
wobei die in Umfangsrichtung durchgehenden Ringe (56) wellenförmig verlaufen, so dass die Umfangszellen (80) so geformt sind, dass sie jeweilige Höhen (58) und Tiefen (60) definieren, und
wobei die Höhen (58) und die Tiefen (60) im Wesentlichen halbkreisförmig sind, einen ersten durchschnittlichen Krümmungsradius aufweisen und so geformt sind, dass sie jeweilige Spannungsverteilungsmerkmale (61) definieren, die einen zweiten durchschnittlichen Krümmungsradius aufweisen, der gleich zwischen 10 % und 50 % des ersten durchschnittlichen Krümmungsradius ist.

10. Vorrichtung nach einem der Ansprüche 1-9,
wobei die Ausbuchtung (42) eine erste Ausbuchtung (42A) ist,
wobei die Fluidströmungsführung (30) an der ersten Teilmenge (32) der Streben (20), der zweiten Teilmenge (34) der Streben (20) und einer dritten Teilmenge (44) der Streben (20) befestigt ist, um das Lumen (36) zu definieren, wobei die erste, die zweite und die dritte Teilmenge (32, 34, 44) keine gemeinsamen Streben (20) enthalten, und
wobei die Streben (20) der dritten Teilmenge (44) elastisch sind, sodass, wenn das Stentgraft (10, 110, 210, 310) den radial ausgedehnten Zustand annimmt:
die Streben (20) der dritten Teilmenge (44) bewirken, dass die Fluidströmungsführung (30) eine zweite Ausbuchtung (42B) definiert, die einen größten Ausbuchtungsradius von der Mittellängsachse (38) definiert, wobei der größte Ausbuchtungsradius mindestens 5 % größer als ein durchschnittlicher Radius der im Wesentlichen zylindrischen röhrenförmigen Abschnitte (40) proximal und distal angrenzend an die zweite Ausbuchtung (42B) ist,
die Streben (20) der dritten Teilmenge (44) Spitzenabschnitte (50) definieren und
die Anzahl der Spitzenabschnitte (50) der Streben (20) der dritten Teilmenge (44), die die zweite Ausbuchtung (42B) definieren, mindestens 50 % größer als die durchschnittliche Anzahl von Umfangszellen (80) in den zwei in Umfangsrichtung durchgehenden Ringen (56) proximal und distal angrenzend an die zweite Ausbuchtung (42B) ist.

11. Vorrichtung nach einem der Ansprüche 1-9, wobei mindestens eine erste der Streben (20) der zweiten Teilmenge (34) mit mindestens einer zweiten der Streben (20) der ersten Teilmenge (32) strukturell einstückig ist.

12. Vorrichtung nach Anspruch 11,
wobei alle Streben (20) der zweiten Teilmenge (34) mit mindestens einer der Streben (20) der ersten Teilmenge (32) strukturell einstückig sind,
wobei keine der Streben (20) der zweiten Teilmenge (34) direkt mit einer der anderen Streben (20) der zweiten Teilmenge (34) verbunden ist, und
wobei keine der Streben (20) der zweiten Teilmenge (34) indirekt mit einer der anderen Streben (20) der zweiten Teilmenge (34) durch andere Streben (20) als die Streben (20) der ersten Teilmenge (32) verbunden ist.

13. Vorrichtung nach einem der Ansprüche 1-9,
wobei die Streben (20) der ersten Teilmenge (32) in einer Vielzahl wellenförmiger, in Umfangsrichtung durchgehender Ringe (56) angeordnet sind, die abwechselnde Höhen (58) und Tiefen (60) aufweisen, und
wobei eine erste Vielzahl (62) der Streben (20) der zweiten Teilmenge (34) ihren Ursprung in einer proximalen Hälfte eines der wellenförmigen, in Umfangsrichtung durchgehenden Ringe (56) hat und eine zweite Vielzahl (64) der Streben (20) der zweiten Teilmenge (34) ihren Ursprung in einer distalen Hälfte des einen der wellenförmigen, in Umfangsrichtung durchgehenden Ringe (56) hat.

14. Vorrichtung nach Anspruch 13,
wobei die erste Vielzahl (62) der Streben (20) der zweiten Teilmenge (34) ihren Ursprung an den am weitesten proximal gelegenen Stellen des einen der wellenförmigen, in Umfangsrichtung durchgehenden Ringe (56) hat, und
wobei die zweite Vielzahl (64) der Streben (20) der zweiten Teilmenge (34) ihren Ursprung an den am weitesten distal gelegenen Stellen des einen der wellenförmigen, in Umfangsrichtung durchgehenden Ringe (56) hat.

15. Vorrichtung nach einem der Ansprüche 1-9,
wobei die Fluidströmungsführung (30) an der ersten Teilmenge (32) der Streben (20), der zweiten Teilmenge (34) der Streben (20) und einer dritten Teilmenge (70) der Streben (20) befestigt ist, um das Lumen (36) zu definieren, wobei die erste, die zweite und die dritte Teilmenge (32, 34, 70) keine gemeinsamen Streben (20) enthalten, und
wobei die Streben (20) der dritten Teilmenge (70) elastisch sind, sodass, wenn das Stentgraft (10, 110, 210, 310) den radial ausgedehnten Zustand annimmt, die Streben (20) der dritten Teilmenge (70) bewirken, dass die Fluidströmungsführung (30) einen aufgeweiteten axialen Abschnitt (72) definiert, der sich zu einem Ende (74) der Fluidströmungsführung (30) erstreckt, wobei der aufgeweitete axiale Abschnitt (72) (a) einen größten aufgeweiteten Radius von der Mittellängsachse (38) aufweist, wobei der größte aufgeweitete Radius mindestens 5 % größer als ein Radius des im Wesentlichen zylindrischen röhrenförmigen Abschnitts (40), der axial an den aufgeweiteten axialen Abschnitt (72) angrenzt, ist, und (b) eine axiale Länge gleich zwischen 5 % und 20 % einer Differenz zwischen (i) dem größten aufgeweiteten Radius und (ii) dem Radius des im Wesentlichen zylindrischen röhrenförmigen Abschnitts (40), der axial an den aufgeweiteten axialen Abschnitt (72) angrenzt, ist.

16. Vorrichtung nach einem der Ansprüche 1-9, wobei, wenn das endovaskuläre Stentgraft (10, 110, 210, 310) im radial komprimierten Zustand entfernbar in dem Abgabekatheter angeordnet ist, die Streben (20) der ersten Teilmenge (32) nicht mit den Streben (20) der zweiten Teilmenge (34) zusammenfallen.

## Revendications

1. Appareil destiné à être utilisé avec un cathéter d'administration, l'appareil comprenant un stent-greffon endovasculaire (10, 110, 210, 310), qui est conçu pour être initialement positionné dans le cathéter d'administration dans un état de compression radiale, et pour adopter un état d'extension radiale lors de son déploiement depuis le cathéter d'administration, et qui comprend :
des entretoises (20) ; et
un guide d'écoulement de fluide (30), qui comprend au moins un tissu biologiquement compatible sensiblement imperméable au sang, et qui est fixé à des premier et deuxième sous-ensembles (32, 34) des entretoises (20), lesdits premier et deuxième sous-ensembles (32, 34) ne comportant pas d'entretoises communes (20),
lesdites entretoises (20) des premier et deuxième sous-ensembles (32, 34) étant élastiques de sorte que, lorsque le stent-greffon (10, 110, 210, 310) adopte l'état d'extension radiale :
le guide d'écoulement de fluide (30) définisse une lumière (36) possédant un axe longitudinal central (38),
les entretoises (20) du premier sous-ensemble (32) soient agencées sous la forme d'une pluralité de cellules circonférentielles (80) dans des anneaux circonférentiellement continus (56), qui amènent le guide d'écoulement de fluide (30) à définir une pluralité de parties tubulaires sensiblement cylindriques (40), et
les entretoises (20) du deuxième sous-ensemble (34) amènent le guide d'écoulement de fluide (30) à définir un renflement (42) présentant le rayon de renflement le plus grand à partir de l'axe longitudinal central (38), ledit rayon de renflement le plus grand étant au moins 5 % supérieur au rayon moyen des parties tubulaires sensiblement cylindriques (40) adjacentes de manière proximale et distale au renflement (42), **caractérisé en ce que** :
les entretoises (20) du deuxième sous-ensemble (34) définissent des parties de pointe (50), et
le nombre des parties de pointe (50) des entretoises (20) du deuxième sous-ensemble (34) qui définissent le renflement (42) est au moins 50 % supérieur au nombre moyen de cellules circonférentielles (80) dans les deux anneaux circonférentiellement continus (56) adjacents de manière proximale et distale au renflement (42).

2. Appareil selon la revendication 1, ledit nombre de parties de pointe (50) des entretoises (20) du deuxième sous-ensemble (34) qui définissent le renflement (42) valant entre 175 % et 250 % du nombre moyen de cellules circonférentielles (80).

3. Appareil selon la revendication 2, ledit nombre des parties de pointe (50) des entretoises (20) du deuxième sous-ensemble (34) qui définissent le renflement (42) étant égal à 200 % du nombre moyen de cellules circonférentielles (80).

4. Appareil selon la revendication 1, ledit nombre de parties de pointe (50) des entretoises (20) du deuxième sous-ensemble (34) qui définissent le renflement (42) valant entre 12 et 16.

5. Appareil selon la revendication 1, ledit renflement (42) entourant circonférentiellement complètement le stent-greffon (10, 110, 210, 310).

6. Appareil selon la revendication 1, lesdites parties de pointe (50) étant directement suturées au guide d'écoulement de fluide (30).

7. Appareil selon la revendication 1, lesdites parties de pointe (50) étant formées de manière à définir des caractéristiques atraumatiques respectives (84).

8. Appareil selon la revendication 1, ledit guide d'écoulement de fluide (30) étant agencé de sorte que l'ensemble du tissu définisse la lumière (36) lorsque le stent-greffon (10, 110, 210, 310) adopte l'état d'extension radiale.

9. Appareil selon la revendication 1,
lesdits anneaux circonférentiellement continus (56) ondulant, de sorte que les cellules circonférentielles (80) soient formées de manière à définir des pics (58) et des creux (60) respectifs, et
lesdits pics (58) et lesdits creux (60) étant sensiblement semi-circulaires, présentant un premier rayon de courbure moyen, et étant formés de manière à définir des caractéristiques respectives de répartition des contraintes (61) en présentant un second rayon de courbure moyen valant entre 10 % et 50 % du premier rayon de courbure moyen.

10. Appareil selon l'une quelconque des revendications 1 à 9,
ledit renflement (42) étant un premier renflement (42A),
ledit guide d'écoulement de fluide (30) étant fixé au premier sous-ensemble (32) des entretoises (20), au deuxième sous-ensemble (34) des entretoises (20) et à un troisième sous-ensemble (44) des entretoises (20) de manière à définir la lumière (36), lesdits premier, deuxième et troisième sous-ensembles (32, 34, 44) ne comportant pas d'entretoises communes (20), et
lesdites entretoises (20) du troisième sous-ensemble (44) étant élastiques de sorte que, lorsque le stent-greffon (10, 110, 210, 310) adopte l'état d'extension radiale :
lesdites entretoises (20) du troisième sous-ensemble (44) amenant le guide d'écoulement de fluide (30) à définir un second renflement (42B) présentant le rayon de renflement le plus grand à partir de l'axe longitudinal central (38), lequel rayon de renflement le plus grand étant au moins 5 % supérieur au rayon moyen des parties tubulaires sensiblement cylindriques (40) adjacentes de manière proximale et distale au second renflement (42B),
lesdites entretoises (20) du troisième sous-ensemble (44) définissant des parties de pointe (50), et
ledit nombre des parties de pointe (50) des entretoises (20) du troisième sous-ensemble (44) qui définissent le second renflement (42B) étant au moins 50 % supérieur au nombre moyen de cellules circonférentielles (80) dans les deux anneaux circonférentiellement continus (56) adjacents de manière proximale et distale au second renflement (42B).

11. Appareil selon l'une quelconque des revendications 1 à 9, au moins une première des entretoises (20) du deuxième sous-ensemble (34) faisant structurellement partie intégrante d'au moins une seconde des entretoises (20) du premier sous-ensemble (32).

12. Appareil selon la revendication 11,
toutes les entretoises (20) du deuxième sous-ensemble (34) faisant structurellement partie intégrante d'au moins l'une des entretoises (20) du premier sous-ensemble (32),
aucune des entretoises (20) du deuxième sous-ensemble (34) n'étant directement reliée à l'une quelconque des autres entretoises (20) du deuxième sous-ensemble (34), et
aucune des entretoises (20) du deuxième sous-ensemble (34) n'étant reliée indirectement à l'une quelconque des autres entretoises (20) du deuxième sous-ensemble (34) par des entretoises (20) autres que les entretoises (20) du premier sous-ensemble (32).

13. Appareil selon l'une quelconque des revendications 1 à 9,
lesdites entretoises (20) du premier sous-ensemble (32) étant agencées dans une pluralité d'anneaux circonférentiellement continus ondulés (56) comportant des pics (58) et des creux (60) alternés, et
une première pluralité (62) des entretoises (20) du deuxième sous-ensemble (34) prenant naissance dans une moitié proximale de l'un des anneaux circonférentiellement continus ondulés (56), et une seconde pluralité (64) des entretoises (20) du deuxième sous-ensemble (34) prenant naissance dans une moitié distale de l'un des anneaux circonférentiellement continus ondulés (56).

14. Appareil selon la revendication 13,
ladite première pluralité (62) des entretoises (20) du deuxième sous-ensemble (34) prenant naissance au niveau des sites les plus proximaux de l'un des anneaux circonférentiellement continus ondulés (56), et
ladite seconde pluralité (64) des entretoises (20) du deuxième sous-ensemble (34) prenant naissance au niveau des sites les plus distaux de l'un des anneaux circonférentiellement continus ondulés (56).

15. Appareil selon l'une quelconque des revendications 1 à 9,
ledit guide d'écoulement de fluide (30) étant fixé au premier sous-ensemble (32) des entretoises (20), au deuxième sous-ensemble (34) des entretoises (20) et à un troisième sous-ensemble (70) des entretoises (20) de manière à définir la lumière (36), lesdits premier, deuxième et troisième sous-ensembles (32, 34, 70) ne comportant pas d'entretoises communes (20), et
lesdites entretoises (20) du troisième sous-ensemble (70) étant élastiques de sorte que, lorsque le stent-greffon (10, 110, 210, 310) adopte l'état d'extension radiale, lesdites entretoises (20) du troisième sous-ensemble (70) amènent le guide d'écoulement de fluide (30) à définir une partie axiale évasée (72) qui s'étend jusqu'à une extrémité (74) du guide d'écoulement de fluide (30), ladite partie axiale évasée (72) présentant (a) le rayon évasé le plus grand à partir de l'axe longitudinal central (38), lequel rayon évasé le plus grand étant au moins 5 % supérieur au rayon de la partie tubulaire sensiblement cylindrique (40) axialement adjacente à la partie axiale évasée (72), et (b) une longueur axiale valant entre 5 % et 20 % de la différence entre (i) le rayon évasé le plus grand et (ii) le rayon de la partie tubulaire sensiblement cylindrique (40) axialement adjacente à la partie axiale évasée (72).

16. Appareil selon l'une quelconque des revendications 1 à 9, lorsque le stent-greffon endovasculaire (10, 110, 210, 310) est disposé de manière amovible dans le cathéter d'administration dans l'état de compression radiale, lesdites entretoises (20) du premier sous-ensemble (32) ne coïncidant pas avec les entretoises (20) du deuxième sous-ensemble (34).
